(19) Europäisches Patentamt
European Patent Office
Office européen des brevets

(11) EP 1 622 874 B1

(12) **EUROPEAN PATENT SPECIFICATION**

(45) Date of publication and mention of the grant of the patent:
**03.10.2007 Bulletin 2007/40**

(21) Application number: **04760215.6**

(22) Date of filing: **16.04.2004**

(51) Int Cl.:
***C07D 215/22*** *(2006.01)* ***C07D 409/04*** *(2006.01)*
***A61K 31/4725*** *(2006.01)* ***A61P 25/00*** *(2006.01)*

(86) International application number:
**PCT/US2004/009290**

(87) International publication number:
**WO 2004/096773 (11.11.2004 Gazette 2004/46)**

(54) **3,4-DIHYDRO-1H-QUINOLIN-2-ONE DERIVATIVES AS NOREPINEPHRINE REUPTAKE INHIBITORS**

3,4-DIHYDRO-1H-CHINOLIN-2-ONDERIVATE ALS NOREPINEPHRIN-WIEDERAUFNAHME-HEMMERN

DERIVES DE 3,4-DIHYDRO-1H-QUINOLIN-2-ONE COMME INHIBITEURS DE LA RECAPTURE DE LA NOREPINEPHRINE

(84) Designated Contracting States:
**AT BE BG CH CY CZ DE DK EE ES FI FR GB GR HU IE IT LI LU MC NL PL PT RO SE SI SK TR**

(30) Priority: **25.04.2003 GB 0309440**
**10.06.2003 US 477277 P**

(43) Date of publication of application:
**08.02.2006 Bulletin 2006/06**

(73) Proprietor: **ELI LILLY AND COMPANY**
**Indianapolis, Indiana 46285 (US)**

(72) Inventors:
- **CAMP, Nicholas, Paul**
  **Basingstoke Hampshire RG21 2XA (GB)**
- **PENARIOL, Roberta**
  **Basingstoke Hampshire RG21 2XA (GB)**
- **BEADLE, Christopher, David**
  **Basingstoke Hampshire RG21 2XA (GB)**

(74) Representative: **Suarez-Miles, Ana Sanchiz et al**
**Eli Lilly and Company Limited**
**Lilly Research Centre**
**Erl Wood Manor**
**Sunninghill Road**
**Windlesham, Surrey GU20 6PH (GB)**

(56) References cited:
**EP-A- 0 919 236**
**WO-A-01/27068**
**WO-A-02/40006**
**WO-A-02/094262**
**WO-A-20/04045718**
**US-A- 5 306 719**
**US-A- 5 552 429**

- **JOURNAL OF MEDICINAL CHEMISTRY, vol. 15, no. 7, 1972, pages 762-770, XP002289097**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 622 874 B1

Printed by Jouve, 75001 PARIS (FR)

## Description

This invention relates to novel quinolone compounds, and to their use in selectively inhibiting norepinephrine reuptake.

Selective inhibition of norepinephrine reuptake is a relatively new mode of action for the treatment of affective disorders. Norepinephrine appears to play an important role in the disturbances of vegetative function associated with affective, anxiety and cognitive disorders. Atomoxetine hydrochloride is a selective inhibitor of norepinephrine, and is marketed for the treatment of attention deficit hyperactivity disorder (ADHD). Reboxetine is a marketed selective norepinephrine reuptake inhibitor for the treatment of depression. A number of 3-(ω-aminoalkyl)-1-phenyl-2-indolinones and the corresponding 1-phenylindolines which were synthesized and evaluated pharmacologically as potential anti-depressant agents are reported in J. of Medicinal Chemistry, vol. 15 (7), 1972, 762-770. US 5,552,429, reports that the power of fluoxetine, venlafaxine, milnacipran and duloxetine to increase the availability of serotonin, norepinephrine and dopamine is augmented by administration in combination with a serotonin 1A receptor agonist. EP 0 919 236, discloses the use of a norepinephrine uptake inhibitor for the treatment of oppositional defiant disorder. WO 01/27068, describes a group of biaryl ether derivatives with activity as serotonin, nonrepinephrine and dopamine reuptake inhibitors which can be used in the treatment of central nervous system and other disorders. WO 02/094262 discloses heteroaryloxy 3-substituted propanamines and their use in inhibiting serotonin and norepinephrine reuptake. WO 02/40006, teaches the use of selective norepinephrine reuptake inhibitors to treat anxiety disorders, especially obsessive-compulsive disorder.

According to the present invention there is provided a compound of formula (I)

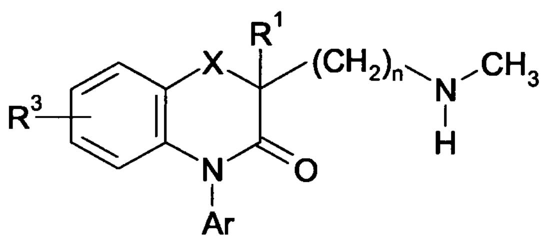

(I)

wherein

-X- is -C($R^4R^5$)-, -O- or -S-;
n is 2 or 3;
$R^1$ is H or $C_1$-$C_4$ alkyl;
$R^3$ is H, halo, $C_1$-$C_4$ alkyl, O($C_1$-$C_4$ alkyl), nitrile, phenyl or substituted phenyl;
$R^4$ and $R^5$ are each independently selected from H or $C_1$-$C_4$alkyl;
Ar- is selected from the group consisting of

(i) 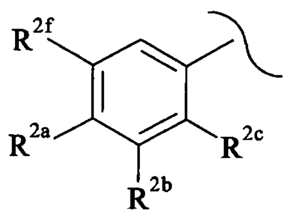 and (ii) 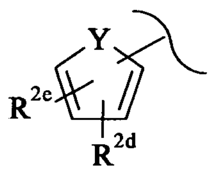

in which
$R^{2a}$ is H, halo, methyl or ethyl;
$R^{2b}$ is H, halo or methyl;
$R^{2c}$ is H, halo, methyl, trifluoromethyl, nitrile, or methoxy;
$R^{2d}$ is H, halo, methyl or ethyl;
$R^{2e}$ is H, halo, methyl, trifluoromethyl, nitrile, or methoxy;
$R^{2f}$ is H, or fluoro;

-Y- is -O-, -S- or -N($R^6$)-; and
$R^6$ is H or methyl
and pharmaceutically acceptable salts thereof.

The term "$C_1$-$C_4$ alkyl" as used herein includes straight and branched chain alkyl groups of 1, 2, 3 or 4 carbon atoms. Thus the term "$C_1$-$C_4$ alkyl" includes methyl, ethyl, n-propyl, isopropyl, n-butyl, isobutyl, sec-butyl and tert-butyl. $C_1$-$C_2$ alkyl groups are preferred. A particularly preferred $C_1$-$C_4$ alkyl group is methyl or ethyl.

The term "halo" includes F, Cl, Br and I, and is preferably F or Cl.

The term "substituted phenyl" means phenyl substituted with 1, 2, 3, 4 or 5 substituents, preferably with 1 or 2, for example 1, substituent. Suitable substituents include $C_1$-$C_4$ alkyl, O($C_1$-$C_4$ alkyl), S($C_1$-$C_4$ alkyl), halo, and phenyl optionally substituted with, for example, $C_1$-$C_4$ alkyl, O($C_1$-$C_4$ alkyl), S($C_1$-$C_4$ alkyl), or halo.

The terms "O($C_1$-$C_4$ alkyl)" or "S($C_1$-$C_4$ alkyl)" mean a $C_1$-$C_4$ alkyl group as defined above linked to the point of substitution via an oxygen or a sulphur atom. An O($C_1$-$C_4$ alkyl) or S($C_1$-$C_4$ alkyl) group includes for example methoxy, ethoxy, thiomethyl or thioethyl.

The present invention includes the pharmaceutically acceptable salts of the compounds of formula (I), formula (Ia) or formula (II). Suitable salts include acid addition salts, including salts formed with inorganic acids, for example hydrochloric, hydrobromic, nitric, sulphuric or phosphoric acids, or with organic acids, such as organic carboxylic or organic sulphonic acids, for example, acetoxybenzoic, citric, glycolic, mandelic-1, mandelic-dl, mandelic-d, maleic, mesotartaric monohydrate, hydroxymaleic, fumaric, lactobionic, malic, methanesulphonic, napsylic, naphthalenedisulfonic, naphtoic, oxalic, palmitic, phenylacetic, propionic, pyridyl hydroxy pyruvic, salicylic, stearic, succinic, sulfanilic, tartaric-1, tartaric-dl, tartaric-d, 2-hydroxyethane sulphonic, toluene-p-sulphonic, and xinafoic acids.

In addition to the pharmaceutically acceptable salts, other salts may serve as intermediates in the purification of compounds or in the preparation of other, for example pharmaceutically acceptable, acid addition salts, or are useful for identification, characterisation or purification.

It will be appreciated that compounds of formula (I), formula (Ia) and formula (II) possess asymmetric carbon atoms, and that in the present invention specific individual stereoisomers are preferred.

A preferred group of compounds according to the present invention is represented by the formula (Ia)

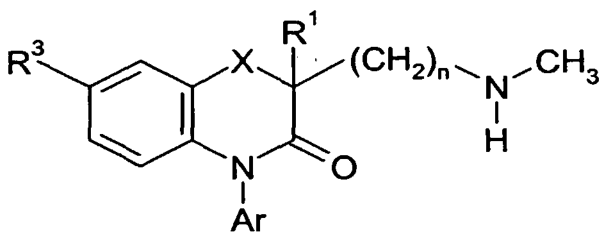

(Ia)

wherein -X-, n, $R^1$, $R^3$ and Ar have the values as defined for formula (I) above.

All the compounds of formulae (I) and (Ia) are embodiments of the present invention, but compounds wherein -X- is -C($R^4R^5$)- are preferred. Even more preferred are compounds wherein X- is -C($R^4R^5$)- and $R^4$ and $R^5$ are both H or $R^4$ and $R^5$ are both the same $C_1$-$C_4$ alkyl.

As mentioned above, all the compounds of formulae (I) and (Ia) above are embodiments of the present invention, but compounds wherein Ar is (i) are also preferred. Preferably Ar is (i) and $R^{2c}$ is H. Even more preferred are compounds wherein Ar is (i), $R^{2c}$ is H, and (a) $R^{2a}$ is H or methyl, $R^{2b}$ is H and $R^{2f}$ is H or (b) $R^{2a}$ is H, $R^{2b}$ is halo, preferably fluoro or chloro and $R^{2f}$ is H or fluoro.

Another group of preferred compounds of the invention are compounds wherein Ar is (ii) and -Y- is -S-. More preferably Ar is 2-thiophenyl or 3-thiophenyl.

A further preferred group of compounds according to the present invention is represented by the formula (II)

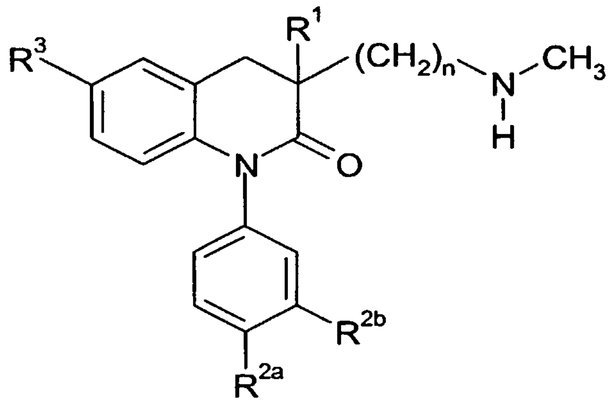

(II)

wherein
n is 2 or 3;
$R^1$ is H or $C_1$-$C_4$ alkyl;
$R^3$ is H, halo, phenyl or substituted phenyl;
$R^{2a}$ is H, halo, methyl or ethyl;
$R^{2b}$ is H, halo or methyl; and pharmaceutically acceptable salts thereof.

It will be appreciated that all the compounds of formulae (I), (Ia) and (II) are embodiments of the present invention, but certain compounds are preferred.

Preferably n is 3.

Also preferably $R^1$ is H, methyl, ethyl or n-propyl.

It is also preferred that $R^3$ is H or halo.

Compounds of the present invention may be prepared using the following methods. General schemes outlining the synthetic routes used to prepare racemic products are given below. All active racemates were separated into single enantiomers using chiral HPLC and in most cases the enantiomers were converted into D-tartrate salts.

Compounds of formula (I) wherein Ar is (i) and $R^{2c}$ is H may be prepared as shown in method A below.

Method A

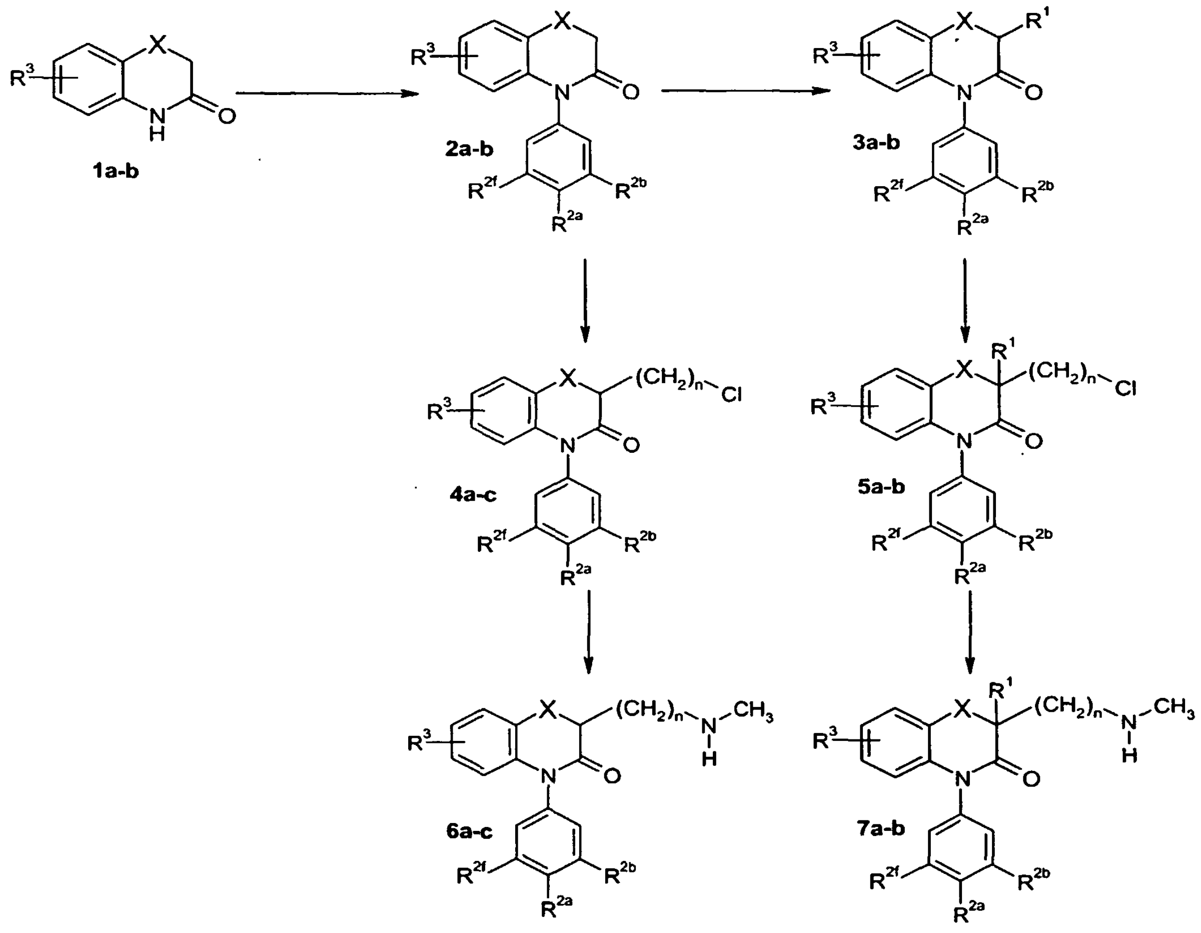

**Scheme 1**

Quinolin-2-one (1) or its corresponding 4-oxo and 4-thio derivatives can be N-arylated using modified conditions to those reported by Buchwald, (J. Am. Chem. Soc., 123, 2001, p. 7727). For example the quinolin-2-one (1) is reacted with 3 equivalents of Ar-Br wherein Ar is (i) and $R^{2c}$ is H, 0.2 equivalents of transcyclohexanediamine, 0.2 equivalent of copper iodide (CuI), 2.1 equivalents of potassium carbonate ($K_2CO_3$), in an organic solvent such as 1,4-dioxane at a temperature of 125°C overnight. The resulting N-arylated quinolin-2-one (2) can be alkylated by treatment with a strong base such as lithium hexamethyldisilazide (LiHMDS) at temperatures of -78°C in a suitable organic solvent such as tetrahydrofuran (THF), followed by the addition of an alkyl halide such as alkyl iodide to give the corresponding 3-alkylated-N-arylated quinolin-2-one derivative (3). Using the same alkylating conditions above with a 1,2-dihaloethane, such as 1-bromo-2-chloroethane, or a 1,3-dihalopropane, such as 1-bromo-3-chloropropane, as alkylating agents provides (4) or (5) wherein n is 2 or 3 respectively. These halo analogues were chosen as ideal precursors to the desired amine products. For instance, treatment of (4) or (5) with aqueous methylamine, in the presence of a catalytic amount of a suitable iodide, such as potassium iodide (KI), in ethanol at 100°C provided the racemic amine products (6) and (7) respectively, in moderate yields.

Compounds of formula (I) wherein Ar is (i), $R^{2c}$ is H and n is 3 may be prepared using alternative method B.

**Method B**

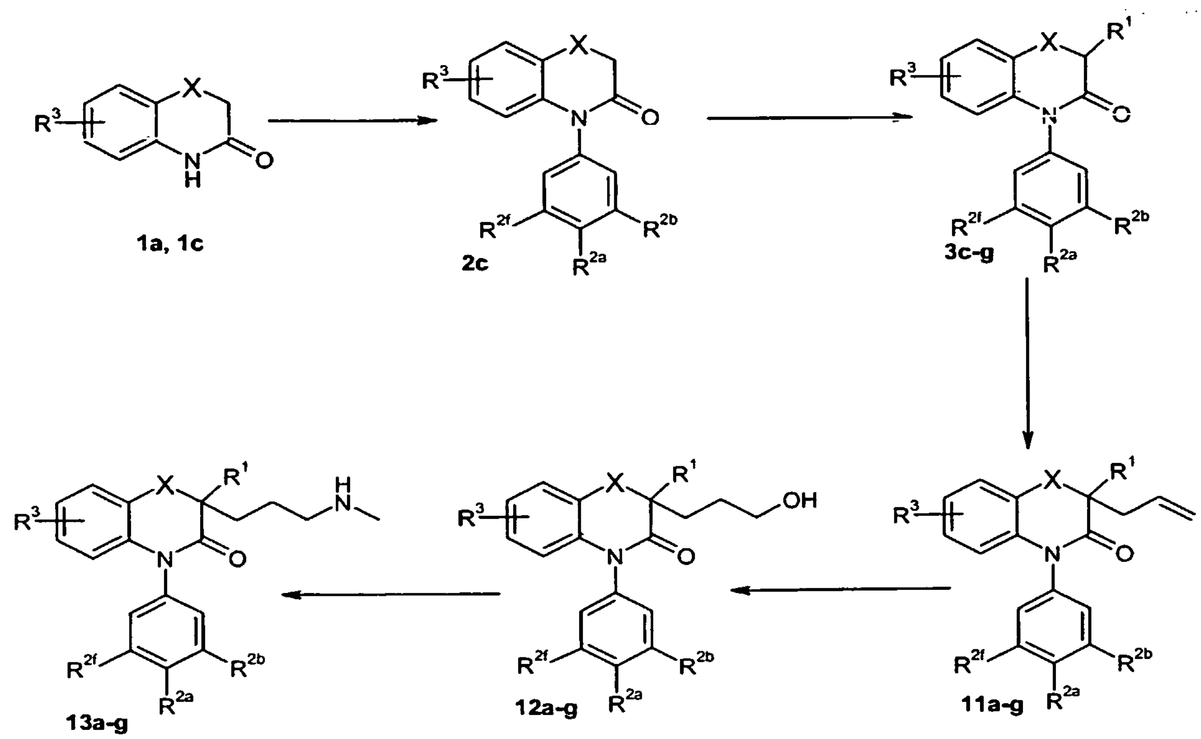

1a, 1c

2c

3c-g

13a-g

12a-g

11a-g

**Scheme 2**

Quinolin-2-ones **(2)** and **(3)** can be alkylated using the aforementioned alkylating procedure using an allyl halide e.g. allyl bromide as the alkylating agent to give the corresponding 3-allyl-N-arylated-quinolin-2-ones **(11a-g).** Said allyl analogues could then be converted to the corresponding primary alcohols **(12a-g)** by a hydroboration procedure involving a suitable borane, such as 9-BBN in a suitable solvent such as THF. Oxidative work up using for example reaction conditions such as aqueous hydrogen peroxide in a solvent such as ethanol, in the presence of a suitable base, such as sodium hydroxide, gave moderate to good yields of alcohol products after column chromatography purification. The alcohols were cleanly converted into their mesylates, by reaction of a mesyl halide such as mesyl chloride in the presence of a suitable base such as triethylamine in a suitable solvent such as THF at a suitable temperature such as 0°C to room temperature. The resulting mesylates are used directly in the amination step described above in method A to provide good yields of the final racemic targets (**13a-g**).

In order to prepare a range of N-arylated analogues advanced intermediates were prepared that could undergo N-arylations with a range of substituted aryl halides, such as aryl bromides or iodides, 2 and 3-halothiophenes, 2 and 3-halofurans or 2 and 3-halopyrroles (Method C). The synthetic route used to prepare intermediates **(19a-b)** is shown below **(Scheme 3**).

**Method C**

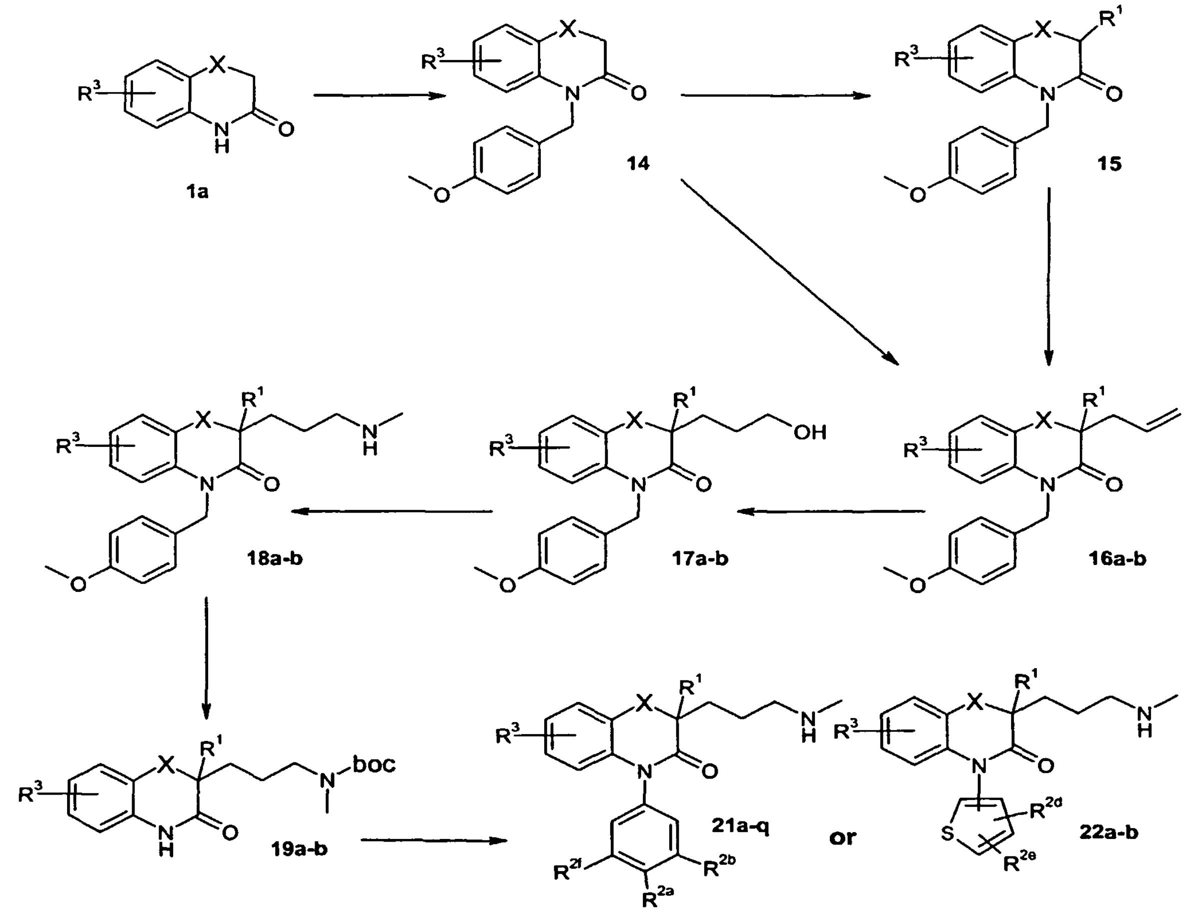

1a
14
15
16a-b
17a-b
18a-b
19a-b
21a-q
or
22a-b

**Scheme 3**

Compounds of formula (I) wherein n is 3 may be prepared as shown in method C. This method is particularly suitable for compounds wherein Ar is (i) and $R^{2c}$ is H or Ar is (ii), wherein -Y- is -S-.

Quinolin-2-one **(1)** can be protected using a suitable amide-protecting group as those described in T.W. Greene, "Protective Groups in Organic Synthesis", John Wiley and Sons, New York, N.Y., 1991, hereafter referred to as "Greene". For example quinolin-2-one (**1**) can be protected with a 4-methoxybenzyl group. The protection reaction can be carried out for example using a suitable base, such as sodium hydride in a suitable solvent, such as dimethylformamide, followed by reaction with a 4-methoxybenzyl halide, such as 4-methoxybenzyl chloride, to give the corresponding N-protected derivative **(14)** in good yield. This intermediate can be converted directly to the allyl analogue (**16a**), wherein $R^1$ = H, in a manner described earlier or converted into the alkyl analogue **(15)** which can be subsequently alkylated with a allyl halide to give the allyl analogue **(16b),** wherein $R^1$ is $C_1$-$C_4$ alkyl. Using the same hydroboration, mesylation and amination sequence described in Method B provided both amines **(18a-b).** Deprotection of protected quinolin-2-one could be achieved using any suitable deprotection conditions as those shown in Greene. For example, the 4-methoxybenzyl group could be cleaved cleanly using trifluoroacetic acid and anisole at 65°C. The resultant product could be selectively protected on the secondary amine with a suitable nitrogen protecting group as those described in Greene. For example, the secondary amine can be protected with a Boc group. The reaction can be carried out with Boc anhydride in a suitable solvent such as THF to provide multi gram quantities of **(19a-b).** Reaction of **(19a-b)** with various aryl bromides using the previously described N-arylation conditions, deprotection using suitable deprotecting conditions such as those described in Greene gave a range of final racemic targets **(21a-q** or **22a-b).** For example, for compounds protected with a Boc group they can be deprotected in the presence of trifluoroacetic acid (TFA) in a suitable organic solvent such as dichoromethane (DCM).

Intermediates **(19 a-b)** wherein $R^3$ is a halo group, for example chloro or bromo, can be used to provide compounds of formula (I) wherein $R^3$ is a phenyl group, such as compound **(24),** via a Suzuki coupling, see scheme 4 below.

**Method D**

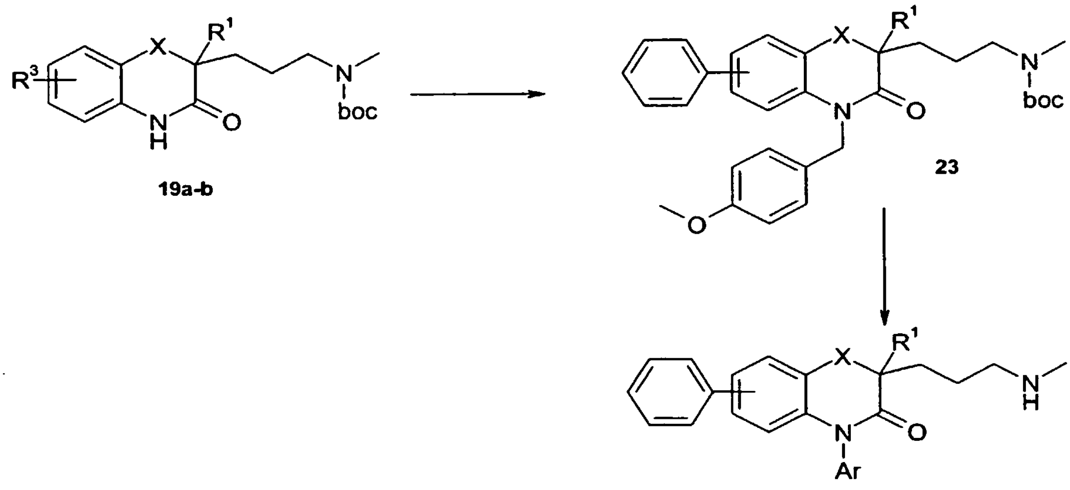

19a-b

23

24

**Scheme 4**

Intermediates **(19a-b),** wherein $R^3$ is for example bromo can be N-protected with a suitable amide protecting group for example 4-methoxybenzyl as described in method C above and then coupled with phenylboronic acid under Suzuki conditions to provide the phenyl analogues **(23).** Deprotection of the 4-methoxybenzyl group with TFA, followed by protection of the resulting secondary amine with a suitable nitrogen protecting group such as Boc followed by subsequent N-arylation and Boc deprotection using the previously described methodology gave the final target **(24).**

It will be appreciated that compounds of formula (Ia) wherein $R^3$ is bromo or chloro can be prepared as shown in methods A to D above starting from the corresponding haloquinolin-2-ones. Alternatively, they can be prepared from the corresponding quinolin-2-one **(1a)** wherein $R^3$ is hydrogen as mentioned above including an extra step comprising the halogenation of a suitable intermediate at some stage of the synthesis. For example quinolin-2-one **(1a)** in method B can be halogenated using N-chlorosuccinimide in a suitable solvent such as DMF at a suitable temperature such as room temperature to give the corresponding 6-chloro-quinolin-2-one (1c) wherein $R^3$ is Cl.

Alternatively intermediates **(19 a-b)** wherein $R^3$ is H in method C can be halogenated in the presence of N-chloro and N-bromosuccinimide in a suitable solvent such as DMF to give the corresponding 6-chloro and 6-bromoquinolin-2-ones **(20a-c).**

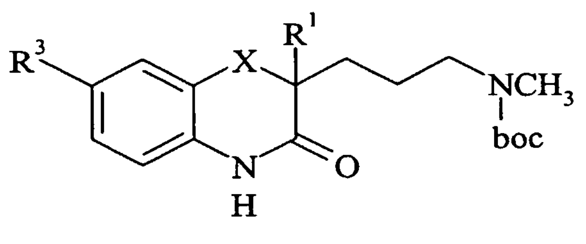

**20a-c**

It will be appreciated that methods A to D above relate to methods for the preparation of compounds of formula I wherein Ar is (i) and $R^{2c}$ is hydrogen. Compounds of formula I wherein Ar is (i) and $R^{2c}$ can be other than hydrogen, can be prepared using any of the general methods mentioned above, starting from the corresponding N-arylated quinolin-2-one **(27).** A general method for preparing said intermediates is illustrated in Scheme 5. Commercially available 3-(2-Bromophenyl)-propionic acids **(25)** can be converted to amide **(26)** using standard amide coupling conditions and converted to the N-arylated quinolin-2-ones **(27)** by an intramolecular, palladium catalysed cyclisation according to the method of Buchwald et al (Tetrahedron, 1996, 52, p. 7525).

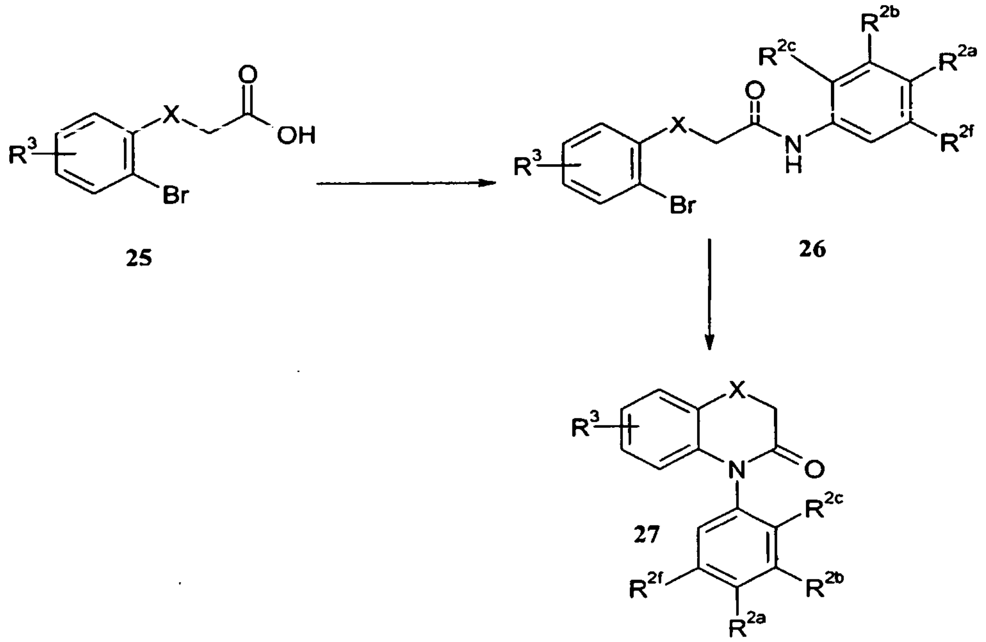

25

26

27

**Scheme 5**

The present invention provides a process for the preparation of a compound of formula (I) comprising reacting methylamine with a compound of formula

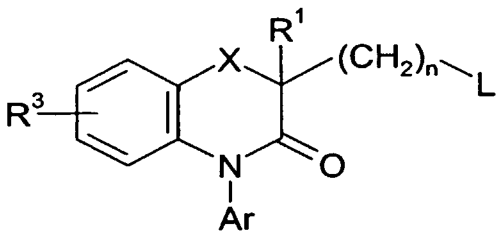

**(III)**

wherein $R^1$, $R^3$, X, n and Ar have the values defined for formula I above and L is a suitable leaving group such as for example chloride, bromide, iodide or mesylate. The reaction can be carried out as described above, by reacting a compound of formula (III) with methylamine for example in the form of aqueous methylamine, optionally in the presence of a catalytic amount of a suitable iodide, such as potassium iodide (KI), in ethanol at 100°C provided the racemic amine products **(6)** and **(7)** respectively, in moderate yields. An optional additional step comprises formation of a pharmaceutically acceptable salt of the compound of formula (1).

The present invention provides a further process for the preparation of a compound of formula (I) comprising the N-deprotection of a compound of formula

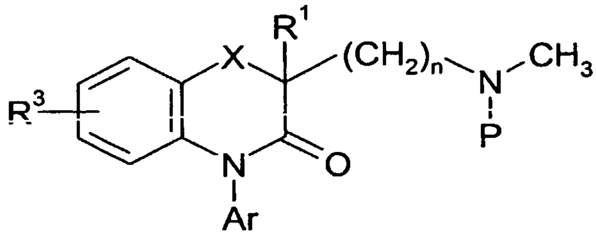

(IV)

wherein $R^1$, $R^3$, X, n and Ar have the values defined for formula I above and P is a suitable nitrogen protecting group such as those described in Greene, for example a Boc group. The reaction is carried out using suitable deprotecting conditions such as those described in Greene according to the nature of the nitrogen-protecting group used (P). For example, for compounds protected with a Boc group they can be deprotected in the presence of trifluoroacetic acid (TFA) in a suitable organic solvent such as dichoromethane (DCM). An optional additional step comprises formation of a pharmaceutically acceptable salt of the compound of formula (I).

Compounds of the present invention are norepinephrine reuptake inhibitors and are selective over other neurotransmitters, such as dopamine or serotonin, that is their binding affinity at the norepinephrine transporter is higher than their affinity for other transporters or other receptors. In addition, they are acid stable.

Thus, the present invention provides a compound of formula (I), formula (Ia) or formula (II), or a pharmaceutically acceptable salt thereof, for use in therapy; and a compound of formula (I), formula (Ia) or formula (II), or a pharmaceutically acceptable salt thereof, for use as a selective inhibitor of the reuptake of norepinephrine

Further, the present invention also provides a compound of formula (I), formula (Ia) or formula (II), or a pharmaceutically acceptable salt thereof, for selectively inhibiting the reuptake of norepinephrine; and a compound of formula (I), formula (Ia) or formula (II), or a pharmaceutically acceptable salt thereof, for treating disorders associated with norepinephrine dysfunction in mammals; and the use of a compound of formula (I), formula (Ia) or formula (II), or a pharmaceutically acceptable salt thereof, in the manufacture of a medicament for selectively inhibiting the reuptake of norepinephrine; and the use of a compound of formula (I), formula (Ia) or formula (II), or a pharmaceutically acceptable salt thereof, in the manufacture of a medicament for the treatment of disorders associated with norepinephrine dysfunction in mammals, including the disorders listed herein.

Disorders associated with norepinephrine dysfunction in mammals, mentioned above in either the uses or the methods of the present invention, include, for example, nervous system conditions such as those selected from the group consisting of an addictive disorder and withdrawal syndrome, an adjustment disorder, an age-associated learning and mental disorder, anorexia nervosa, apathy, an attention-deficit disorder (ADD) due to general medical conditions, attention-deficit hyperactivity disorder (ADHD), bipolar disorder, bulimia nervosa, chronic fatigue syndrome, chronic or acute stress, conduct disorder, cyclothymic disorder, depression, dysthymic disorder, fibromyalgia and other somatoform disorders, generalized anxiety disorder, incontinence, an inhalation disorder, an intoxication disorder, mania, migraine headaches, obesity, obsessive compulsive disorders and related spectrum disorders, oppositional defiant disorder, panic disorder, peripheral neuropathy, post-traumatic stress disorder, premenstrual dysphoric disorder, a psychotic disorder, seasonal affective disorder, a sleep disorder, social phobia, a specific developmental disorder, selective serotonin reuptake inhibition (SSRI) "poop out" syndrome, TIC disorders, cognitive disorders including mild cognitive impairment (MCI), dementia of the Alzheimers type (DAT), vascular dementia and cognitive impairment associated with schizophrenia (CIAS), hypotensive states including orthostatic hypotension, and pain including chronic pain, neuropathic pain and antinociceptive pain.

In addition to the compounds of formula (I), formula (Ia) and formula (II), and processes for the preparation of said compounds, the present invention further provides pharmaceutical compositions comprising a compound of formula (I), formula (Ia) or formula (II), or a pharmaceutically acceptable salt thereof, together with a pharmaceutically acceptable diluent or carrier.

The compounds of the present invention may be used as medicaments in human or veterinary medicine. The compounds may be administered by various routes, for example, by oral or rectal routes, topically or parenterally, for example by injection, and are usually employed in the form of a pharmaceutical composition.

Such compositions may be prepared by methods well known in the pharmaceutical art and normally comprise at least one active compound in association with a pharmaceutically acceptable diluent or carrier. In making the compositions of the present invention, the active ingredient will usually be mixed with a carrier or diluted by a carrier, and/or enclosed within a carrier which may, for example, be in the form of a capsule, sachet, paper or other container. Where the carrier serves as a diluent, it may be solid, semi-solid, or liquid material which acts as a vehicle, excipient or medium

for the active ingredient. Thus, the composition may be in the form of tablets, lozenges, sachets, cachets, elixirs, suspensions, solutions, syrups, aerosol (as a solid or in a liquid medium), ointments containing, for example, up to 10% by weight of the active compound, soft and hard gelatin capsules, suppositories, injection solutions and suspensions and sterile packaged powders.

Some examples of suitable carriers are lactose, dextrose, vegetable oils, benzyl alcohols, alkylene glycols, polyethylene glycols, glycerol triacetate, gelatin, carbohydrates such as starch and petroleum jelly, sucrose sorbitol, mannitol, starches, gum acacia, calcium phosphate, alginates, tragacanth, gelatin, syrup, methyl cellulose, methyl- and propyl- hydrobenzoate, talc, magnesium stearate and mineral oil. The compounds of formula (I) can also be lyophilized and the lyophilizates obtained used, for example, for the production of injection preparations. The preparations indicated can be sterilized and/or can contain auxiliaries such as lubricants, preservatives, stabilizers and/or wetting agents, emulsifiers, salts for affecting the osmotic pressure, buffer substances, colourants, flavourings and/or one or more further active compounds, e.g. one or more vitamins. Compositions of the invention may be formulated so as to provide, quick, sustained or delayed release of the active ingredient after administration to the patient by employing procedures well known in the art.

The compositions are preferably formulated in a unit dosage form, each dosage containing from about 5 to about 500 mg, more usually about 25 to about 300 mg, of the active ingredient. The term "unit dosage form" refers to physically discrete units suitable as unitary doses for human subjects and other mammals, each unit containing a predetermined quantity of active material calculated to produce the desired therapeutic effect, in association with a suitable pharmaceutical carrier.

The following examples illustrate particular embodiments of compounds of the present invention and methods for their preparation.

**Method A**

**Preparation of Intermediates**

**1-Phenyl-3,4-dihydro-*1H*-quinolin-2-one (2a)**

A stirred mixture of 3,4-Dihydro-*1H*-quinolin-2-one **(1a)** (1.47 g. 10 mmol), $K_2CO_3$ (2.9 g, 21 mmol), *trans*-cyclohexane-1,2-diamine (240 μL, 2 mmol) and bromobenzene (3.16 mL, 30 mmol) in 1,4-dioxane (10 mL) was heated under a nitrogen atmosphere at 125°C for 5 min to deoxygenate the reaction mixture. Copper (I) iodide (380 mg, 2 mmol) was added in one portion and the reaction mixture was refluxed overnight at 125°C. After cooling to rt, the reaction mixture was poured into ethyl acetate (100 mL) and extracted with water. The organic layer was separated, dried over $MgSO_4$ and concentrated. Treatment of the residue with ether (100 mL) and cooling (ice bath) gave the product as a white solid after filtration (1.77 g, 79%).

**6-Fluoro-1-*p*-tolyl-3,4-dihydro-*1H*-quinolin-2-one (2b)**

This was prepared using the method described for **(2a)** using 6-Fluoro-3,4-dihydro-*1H*-quinolin-2-one **(1b)** (617 mg, 3.7 mmol) and 4-bromotoluene (1.91 g, 11 mmol) to give the crude product, which was purified using automated chromatography (silica) (0 to 60% ethyl acetate\cyclohexane gradient) to provide the product as a light brown solid (880 mg, 92%).

**3-Methyl-1-phenyl-3,4-dihydro-*1H*-quinolin-2-one (3a)**

To a soln of (**2a**) (892 mg, 4 mmol) in anhydrous THF (40 mL) at -78°C under nitrogen was added LiHMDS (4.4 mL, 1M soln in hexanes, 4.4 mmol) dropwise over 10 min. The reaction mixture was left at -78°C for 30 min and then a solution of methyl iodide (298 μL, 4.8 mmol) in THF (1 mL) was added dropwise. The reaction mixture was warmed slowly to rt, quenched with water (2 mL) and extracted with ethyl acetate (100 mL). The organic layer was separated, dried over $MgSO_4$ and concentrated. The residue was purified by column chromatograpy (silica, gradient 100% hexane to ethyl acetate\hexane 3:10) giving the product as an oil (667 mg, 70%).

**3-Ethyl-1-phenyl-3,4-dihydro-*1H*-quinolin-2-one (3b)**

This was prepared in a similar manner to (**3a**) on a 1.5 mmol scale using 1-iodoethane (125 μL, 1.1 eq.) as the alkylating agent. The crude product (378 mg) was used directly in the next step.

**3-(3-Chloro-propyl)-1-phenyl-3,4-dihydro-*1H*-quinolin-2-one (4a)**

To a soln of (2a) (892 mg, 4 mmol) in anhydrous THF (40 mL) at -78°C under nitrogen was added LiHMDS (4.4 mL, 1M soln in hexanes, 4.4 mmol) dropwise over 10 min. The reaction mixture was left at -78°C for 30 min and then a solution of 1-bromo-3-chloropropane (405 μL, 4.4 mmol) in THF (1 mL) was added dropwise. The reaction mixture was warmed slowly to rt, quenched with water (2 mL) and extracted with ethyl acetate (100 mL). The organic layer was separated, dried over $MgSO_4$ and concentrated. The crude product (1.2 g) was used directly in the next step.

**3-(3-Chloro-propyl)-6-fluoro-1-*p*-tolyl-3,4-dihydro-*1H*-quinolin-2-one (4b)**

This was prepared from (**2b**) (300 mg, 1.17 mmol) using the method described for **(4a)** using 1-bromo-3-chloropropane (140 μL, 1.4 mmol) as the alkylating agent. The crude product (399 mg) was used directly in the next step.

**3-(2-Chloro-ethyl)-1-phenyl-3,4-dihydro-*1H*-quinolin-2-one (4c)**

This was prepared from **(2a)** (892 mg, 4.0 mmol) using the method described for **(4a)** using 1-bromo-2-chloroethane (365 μL, 4.4 mmol) as the alkylating agent. The crude product (1 g) was used directly in the next step.

**3-(3-Chloro-propyl)-3-methyl-1-phenyl-3,4-dihydro-*1H*-quinolin-2-one (5a)**

This was prepared from **(3a)** (462 mg, 1.95 mmol) using the method described for **(4a)** using 1-bromo-3-chloropropane (270 μL, 2.7 mmol) as the alkylating agent. The crude product (650 mg) was used directly in the next step.

**3-(3-Chloro-propyl)-3-ethyl-1-phenyl-3,4-dihydro-*1H*-quinolin-2-one (5b)**

This was prepared from **(3b)** (378 mg, 1.5 mmol) using the method described for **(4a)** using 1-bromo-3-chloropropane (179 μL, 1.8 mmol) as the alkylating agent. The crude product (528 mg) was used directly in the next step.

**<u>Examples</u>**

**Example 1: 3-(3-Methylamino-propyl)-1-phenyl-3,4-dihydro-*1H*-quinolin-2-one (6a)**

A soln of **(4a)** (1.2 g, 4 mmol), potassium iodide (200 mg, 1.2 mmol) and aqueous 40% methylamine (12 mL) in ethanol (30 mL) was refluxed at 100°C under nitrogen for 3 h. The reaction mixture was cooled, poured into water and extracted with ethyl acetate (100 mL). The organic layer was separated, dried over $MgSO_4$ and concentrated. The product was purified by preparative LCMS to give 500 mg of the racemate. The racemate was separated into its individual enantiomers using chiral HPLC. $^1H$ NMR (300 MHz, $CDCl_3$) (racemate & isomer) δ 1.5-1.73 (m, 4H), 1.88-1.97 (m, 1H), 2.43 (s, 3H), 2.62 (t, J= 6.69 Hz, 2H), 2.70-2.79 (m, 1H), 2.84-2.92 (m, 1H), 3.15 (dd, J= 15.45, 5.28 Hz, 1H), 6.33 (d, J= 7.73 Hz, 1H), 6.95-7.06 (m, 2H), 7.19-7.22 (m, 3H), 7.38-7.43 (m, 1H), 7.47-7.52 (m, 2H). LCMS (12 minute method) $[M+H]^+$ = 295 @ Rt 4.0 min (100%).

**Example 2: 6-Fluoro-3-(3-methylamino-propyl)-1-*p*-tolyl-3,4-dihydro-1*H*-quinolin-2-one (6b)**

This was prepared in an identical manner to (**6a**) using crude (**4b**) (399 mg) to give the crude product, which was purified by preparative LCMS to give the product (35 mg). $^1H$ NMR (300 MHz, $CDCl_3$) (racemate) δ 1.40-1.70 (m, 3H), 1.75-1.90 (m, 4H), 2.34 (s, 3H), 2.36 (s, 3H), 2.50-2.83 (m, 2H), 3.01-3.08 (m, 1H), 6.21-6.26 (m, 1H), 6.62-6.68 (m, 1H), 6.82-6.86 (m, 1H), 6.99 (d, J= 8.1 Hz, 2H), 7.22 (d, J= 8.1 Hz, 2H). LCMS (12 minute method) $[M+H]^+$ = 327 @ Rt 4.8 min (100%).

**Example 3**: **3-(2-Methylamino-ethyl)-1-phenyl-3,4-dihydro-1H-quinolin-2-one (6c)**

This was prepared in an identical manner to (**6a)** using crude (**4c**) (1g) to give the racemate (80 mg). The racemate was separated into its individual enantiomers using chiral HPLC. $^1H$ NMR (300 MHz, $CDCl_3$) (racemate & isomer) δ ppm 1.64-1.76 (m, 1H), 1.79 (br, 1H), 2.03-2.18 (m, 1H), 2.44 (s, 3H), 2.71-2.82 (m, 2H), 2.82-2.94 (m, 2H), 3.09-3.21 (m, 1H), 6.33 (dd, J= 7.91, 1.32 Hz, 1H), 6.94-7.07 (m, 2H), 7.18-7.24 (m, 3H), 7.37-7.44 (m, 1H), 7.47-7.54 (m, 2H). LCMS (12 minute method) $[M+H]^+$ = 281 @Rt 3.82 min (100%).

**Example 4: 3-Methyl-3-(3-methylamino-propyl)-1-phenyl-3,4-dihydro-*1H*-quinolin-2-one (7a)**

This was prepared in an identical manner to (**6a**) using crude (**5a**) (650 mg) to give the crude product (198 mg), which was purified by preparative LCMS. The purified racemate was then separated into its individual enantiomers using chiral HPLC. $^1$H NMR (300 MHz, $CDCl_3$) (isomer) δ ppm 1.27 (s, 3H), 1.43 (br, 1H), 1.53-1.66 (m, 4H), 2.39 (s, 3H), 2.54 (t, J= 6.12 Hz, 2H), 2.91 (d, J= 15.64 Hz, 1H), 2.98 (d, J= 15.64 Hz, 1H), 6.28 (dd, J= 7.91, 1.32 Hz, 1H), 6.97 (td, J= 7.21, 1.41 Hz, 1H), 7.03 (td, J= 7.68, 1.98 Hz, 1H), 7.14-7.22 (m, 3H), 7.36-7.44 (m, 1H), 7.46-7.53 (m, 2H). LCMS (12 minute method) $[M+H]^+$ = 309 @Rt 4.21 min (100%).

**Example 5: 3-Ethyl-3-(3-methylamino-propyl)-1-phenyl-3,4-dihydro-*1H*-quinolin-2-one (7b)**

This was prepared in an identical manner to **(6a)** using crude **(5b)** (528 mg) to give the crude product (105 mg), which was purified by preparative LCMS. The purified racemate was then separated into its individual enantiomers using chiral HPLC. $^1$H NMR (300 MHz, $CDCl_3$) (racemate) δ 0.93 (t, J= 7.53 Hz, 3H), 1.56-1.75 (m, 6H), 1.91 (bs, 1H), 2.41 (s, 3H), 2.55-2.60 (m, 2H), 2.91 (d, J= 15.82, 1H), 3.02 (d, J= 15.82, 1H), 6.25-6.28 (m, 1H), 6.94-7.05 (m, 2H), 7.16-7.19 (m, 3H), 7.38-7.43 (m, 1H), 7.4-7.52 (m, 2H). $^1$H NMR (300 MHz, MeOD-d4) (isomer D-tartrate salt) δ 0.85 (t, J= 7.53 Hz, 3H), 1.45-1.75 (m, 6H), 2.57 (s, 2H), 2.83-2.89 (m, 2H), 3.01-3.06 (d, J= 16.01, 1H), 4.32 (s, 2H), 6.11-6.14 (m, 1H), 6.89-6.97 (m, 2H), 7.09 (d, J= 7.16 Hz, 2H), 7.15-7.18 (m, 1H), 7.37 (t, J= 7.35 Hz, 1H), 7.46 (t, J= 7.35 Hz, 2H). LCMS (12 minute method) $[M+H]^+$ = 323 @ Rt 4.9 min (98%).

**Method B**

**Preparation of Intermediates**

**1-*p*-Tolyl-3,4-dihydro-*1H* quinolin-2-one (2c)**

A stirred mixture of 3,4-Dihydro-*1H*-quinolin-2-one **(1a)** (4.41 g. 30 mmol), $K_2CO_3$ (8.7 g, 63 mmol), *trans*-cyclohexane-1,2-diamine (720 μL, 2 mmol) and 4-bromotoluene (15.4 g, 90 mmol) in 1,4-dioxane (30 mL) was heated under a nitrogen atmosphere at 125°C for 5 min to deoxygenate the reaction mixture. Copper (I) iodide (1.14 g, 2 mmol) was added in one portion and the reaction mixture was refluxed overnight at 125°C. After cooling to rt, the reaction mixture was filtered through celite, poured into ethyl acetate (100 mL) and extracted with water. The organic layer was separated, dried over $MgSO_4$ and concentrated. Treatment of the residue with ether (200 mL) and cooling (ice bath) gave the product as a white solid after filtration (6.2 g, 87%).

**1-Phenyl-3-propyl-3,4-dihydro-*1H*-quinolin-2-one (3c)**

This was prepared from **(2a)** (669 mg, 3 mmol) and 1-iodopropane (352 μl, 1.2 eq.) as the alkylating agent. The crude product (780 mg) was used directly in the next step.

**3-Ethyl-1-*p*-tolyl-3,4-dihydro-*1H*-quinolin-2-one (3d)**

This was prepared from (**2c**) (711 mg, 3 mmol) and 1-iodoethane (265 μl, 1.2 eq.) as the alkylating agent. The crude product (800 mg) was used directly in the next step.

**3-Propyl-1-*p*-tolyl-3,4-dihydro-*1H* quinolin-2-one (3e)**

This was prepared from (**2c**) (711 mg, 3 mmol) and 1-iodopropane (352 μl, 1.2 eq.) as the alkylating agent. The crude product (840 mg) was used directly in the next step.

**3-Butyl-1-*p*-tolyl-3,4-dihydro-*1H*-quinolin-2-one (3f)**

This was prepared from (**2c**) (711 mg, 3 mmol) and 1-iodobutane (354 μl, 1.1 eq.) as the alkylating agent. The crude product (790 mg) was used directly in the next step.

**3-Isopropyl-1-*p*-tolyl-3,4-dihydro-*1H* quinolin-2-one (3g)**

This was prepared from (**2c**) (711 mg, 3 mmol) and 2-iodopropane (330 μl, 1.1 eq.) as the alkylating agent. The crude product (806 mg) was used directly in the next step.

**3-Allyl-3-ethyl-1-*p*-tolyl-3,4-dihydro-*1H*-quinolin-2-one (11b)**

To a soln of **(3d)** (800 mg, 2.7 mmol) in anhydrous THF (30 mL) at -78°C under nitrogen was added LiHMDS (3 mL, 1M soln in hexanes, 3 mmol) dropwise over 10 min. The reaction mixture was left at -78°C for 30 min and then a solution of allyl bromide (280 μL, 3.2 mmol) in THF (1 mL) was added dropwise. The reaction mixture was warmed slowly to rt, quenched with water (2 mL) and extracted with ethyl acetate (100 mL). The organic layer was separated, dried over $MgSO_4$ and concentrated. The crude product (920 mg) was used directly in the next step.

**3-Ethyl-3-(3-hydroxypropyl)-1-*p*-tolyl-3,4-dihydro-*1H*-quinolin-2-one (12b)**

To a soln of **(11b)** (732 mg, 2.4 mmol) in anhydrous THF (25 mL) at 0°C under nitrogen was added 9-BBN (12 mL, 0.5M soln in THF, 6 mmol, 2.5 eq.) dropwise over 10 min. The reaction mixture was warmed to rt and left to stir overnight. The resultant yellow soln was cooled to 0°C and then quenched carefully with ethanol (3 mL), followed by aq. NaOH (1.8 mL, 3N soln). Finally, aq. $H_2O$ (1.8 mL, 37% soln) was added dropwise maintaining the internal reaction mixture temp between 5 and 10 °C. The reaction mixture was warmed to rt and then refluxed for 90 min. The reaction mixture was cooled to rt, poured into ethyl acetate and water and extracted. The organic layer was separated, dried over $MgSO_4$ and concentrated. The crude product was purified using automated chromatography (silica) (0 to 60% ethyl acetate\cyclohexane gradient) to provide (**12b**) as a clear oil (540 mg, 70%).

## Examples

**Example 6: 3-Ethyl-3-(3-methylamino-propyl)-1-*p*-tolyl-3,4-dihydro-*1H*-quinolin-2-one (13b)**

To a soln of (**12b**) (540 mg, 1.67 mmol) and triethylamine (350 μL, 2.5 mmol) in anhydrous THF (20 mL) at 0°C under nitrogen was added dropwise a soln of methanesulfonyl chloride (142 μL, 1.8 mmol) in THF (1 mL). The reaction mixture was warmed to rt and stirred for 3 h. The reaction mixture was poured into ethyl acetate and water and extracted. The organic layer was separated, dried over $MgSO_4$ and concentrated. The crude mesylate (670 mg, 100%) was dissolved in ethanol (10 mL) and aqueous 40% methylamine (5 mL) and heated at 65°C under nitrogen for 2 h. The reaction mixture was cooled, poured into water and extracted with ethyl acetate (100 mL). The organic layer was separated, dried over $MgSO_4$ and concentrated. The product was purified by SCX-2 to give 384 mg of the racemate. The racemate was separated into its individual enantiomers using chiral HPLC. Each enantiomer was dissolved in $CH_2Cl_2$ (2 mL) and treated with 1 equivalent of D-tartaric acid dissolved in a minimum volume of warm methanol. The resultant soln was concentrated and the solid was dried under vacuo to provide the D-tartrate salt of the amine. $^1$H NMR (300 MHz, $CDCl_3$) (racemate) δ 0.92 (t, J= 7.44 Hz, 3H), 1.49-1.75 (m, 6H), 1.81 (br, 1H), 2.40 (s, 6H), 2.57 (t, J= 6.59 Hz, 2H), 2.89 (d, J= 15.82 Hz, 1H), 3.00 (d, J= 15.82 Hz, 1H), 6.29 (d, J= 7.91 Hz, 1H), 6.92-7.08 (m, 4H), 7.16 (d, J= 7.16 Hz, 1H), 7.29 (d, J= 7.91 Hz, 2H). $^1$H NMR (300 MHz, MeOD-d4) (isomer D-tartrate salt) δ 0.93 (t, J= 7.44 Hz, 3H), 1.54-1.84 (m, 6H), 2.42 (s, 3H), 2.66 (s, 3H), 2.91-3.00 (m, 3H), 3.11 (d, J= 15.83 Hz, 1H), 4.41 (s, 2H), 6.22-6.27 (m, 1H), 6.80-7.07 (m, 4H), 7.21-7.27 (m, 1H), 7.36 (d, J= 7.91 Hz, 2H). LCMS (12 minute method) $[M+H]^+$ = 337 @Rt 5.21 min (100%).

**Example 7: 3-(3-Methylamino-propyl)-1-phenyl-3-propyl-3,4-dihydro-*1H*-quinolin-2-one (13a)**

This was prepared from (**3c**) (780 mg, 2.9 mmol) using the same synthetic sequence described in method B (**3d** to **13b**) to give 233 mg of the racemate. The racemate was separated into its individual enantiomers using chiral HPLC and each enantiomer was converted into its D-tartrate salt as described for **(13b).** $^1$H NMR (300 MHz, $CDCl_3$) (racemate) δ 0.88 (t, J= 7.16 Hz, 3H), 1.26-1.48 (m, 2H), 1.50-1.78 (m, 7H), 2.40 (s, 3H), 2.56 (t, J= 6.59 Hz, 2H), 2.92 (d, J= 15.83 Hz, 1H), 3.01 (d, J= 15.83 Hz, 1H), 6.25-6.28 (m, 1H), 6.94-7.05 (m, 2H), 7.16-7.19 (m, 3H), 7.37-7.42 (m, 1H), 7.47-7.52 (m, 2H). $^1$H NMR (300 MHz, MeOD-d4) (isomer D-tartrate salt) δ 0.77-0.82 (t, J= 7.06 Hz, 3H), 1.24-1.35 (m, 2H), 1.44-1.51 (m, 2H), 1.69 (bs, 3H), 2.56 (s, 3H), 2.84-2.89 (m, 3H), 3.01-3.06 (d, J= 15.83 Hz, 1H), 3.20-3.22 (q, J=1.55 Hz, 2H), 4.30 (s, 2H), 6.11-6.14 (dd, J= 7.72, 2.26 Hz, 1H), 6.89-6.97 (m, 2H), 7.07-7.10 (m, 2H), 7.14-7.17 (m, 1H), 7.34-7.39 (t, J= 7.35 Hz, 1H), 7.43-7.48 (t, J= 7.35 Hz, 2H). LCMS (12 minute method) $[M+H]^+$ = 337 @ Rt 5.2 min (100%).

**Example 8: 3-(3-Methylamino-propyl)-3-propyl-1-*p*-tolyl-3,4-dihydro-*1H*-quinolin-2-one (13c)**

This was prepared from (**3e**) (840 mg, 2.6 mmol) using the same synthetic sequence described in method B (**3d** to **13b**) to give 393 mg of the racemate. The racemate was separated into its individual enantiomers using chiral HPLC and each enantiomer was converted into its D-tartrate salt as described for **(13b).** $^1$H NMR (300 MHz, $CDCl_3$)

(racemate) δ 0.88 (t, J= 7.16 Hz, 3H), 1.20-1.75 (m, 11H), 2.39 (s, 3H), 2.40 (s, 3H), 2.90 (d, J= 15.64 Hz, 1H), 2.99 (d, J= 15.64 Hz, 1H), 6.29 (d, J= 7.72 Hz, 1H), 6.93-7.07 (m, 4H), 7.14-7.16 (m, 1H), 7.25-7.31 (m, 2H). $^1$H NMR (300 MHz, MeOD-d4) (isomer D-tartrate salt) δ 0.91 (t, J= 7.06 Hz, 3H), 1.28-1.85 (m, 8H), 2.44 (s, 3H), 2.68 (s, 3H), 2.94-2.99 (m, 3H), 3.14 (d, J= 15.82 Hz, 1H), 4.41 (s, 2H), 6.25-6.28 (m, 1H), 7.02-7.07 (m, 4H), 7.25-7.28 (m, 1H), 7.38 (d, J= 7.91 Hz, 2H). LCMS (12 minute method) $[M+H]^+$ = 351 @ Rt 5.6 min (100%).

**Example 9: 3-Butyl-3-(3-methylamino-propyl)-1-*p*-tolyl-3,4-dihydro-*1H* quinolin-2-one (13d)**

This was prepared from (**3f**) (790 mg, 2.7 mmol) using the same synthetic sequence described in method B **(3d** to **13b)** to give 334 mg of the racemate. The racemate was separated into its individual enantiomers using chiral HPLC and each enantiomer was converted into its D-tartrate salt as described for **(13b).** $^1$H NMR (300 MHz, $CDCl_3$) (racemate) δ 0.87 (t, J= 6.97 Hz, 3H), 1.20-1.40 (m, 4H), 1.55-1.74 (m, 6H), 2.40 (s, 3H), 2.40 (s, 3H), 2.55 (t, J= 6.78 Hz, 3H), 2.91 (d, J= 15.63 Hz, 1H), 2.99 (d, J= 15.63 Hz, 1H), 6.28-6.31 (m, 1H), 6.93-7.00 (m, 2H), 7.02-7.06 (m, 2H), 7.14-7.16 (m, 1H), 7.29 (d, J= 8.07 Hz, 2H). $^1$H NMR (300 MHz, MeOD-d4) (isomer D-tartrate salt) δ 0.90 (t, J= 6.97 Hz, 3H), 1.20-1.85 (m, 10H), 2.44 (s, 3H), 2.68 (s, 3H), 2.94-2.99 (m, 3H), 3.14 (d, J= 15.82 Hz, 1H), 4.42 (s, 2H), 6.25-6.28 (m, 1H), 7.00-7.07 (m, 4H), 7.25-7.28 (m, 1H), 7.38 (d, J= 7.91 Hz, 2H). LCMS (12 minute method) $[M+H]^+$ = 365 @ Rt 5.9 min (100%).

**Example 10: 3-Isopropyl-3-(3-methylamino-propyl)-1-*p*-tolyl-3,4-dihydro-*1H*-quinolin-2-one (13e)**

This was prepared from **(3g)** (806 mg, 2.89 mmol) using the same synthetic sequence described in method B **(3d** to **13b)** to give 307 mg of the racemate. $^1$H NMR (300 MHz, $CDCl_3$) (racemate) δ ppm 0.92 (dd, J= 8.95, 6.88 Hz, 6H), 1.39-1.88 (m, 5H), 2.12-2.23 (m, 1H), 2.39 (s, 3H), 2.40 (s, 3H), 2.56 (t, J= 6.78 Hz, 2H), 2.94 (d, J= 15.92 Hz, 1H), 3.00 (d, J= 15.92 Hz, 1H), 6.28 (dd, J= 7.82, 1.04 Hz, 1H), 6.92-7.06 (m, 4H), 7.16 (dd, J= 6.97, 1.13 Hz, 1H), 7.29 (d, J= 7.91 Hz, 2H). LCMS (12 minute method) $[M+H]^+$ = 351 @Rt 5.55 min (100%).

**Example 11: 6-Chloro-3-ethyl-3-(3-methylamino-propyl)-1-*p*-tolyl-3,4-dihydro-*1H*-quinolin-2-one (13f)**

This was prepared from **(1c)** using the same synthetic sequence described in method B to give 205 mg of the racemate. The racemate was separated into its individual enantiomers using chiral HPLC and each enantiomer was converted into its D-tartrate salt as described for **(13b).** $^1$H NMR (300 MHz, $CDCl_3$) (racemate) δ ppm 0.91 (t, J= 7.44 Hz, 3H), 1.50-1.75 (m, 6H), 2.15 (br, 1H), 2.40 (s, 3H), 2.41 (s, 3H), 2.55-2.64 (m, 2H), 2.85 (d, J= 16.01 Hz, 1H), 2.97 (d, J= 16.01 Hz, 1H), 6.23 (d, J= 8.85 Hz, 1H), 6.97 (dd, J= 8.67, 2.45 Hz, 1H), 7.02 (d, J= 8.29 Hz, 2H), 7.14 (d, J= 2.26 Hz, 1H), 7.29 (d, J= 8.10 Hz, 2H). $^1$H NMR (300 MHz, MeOD-d4) (isomer, D-tartrate salt) δ ppm 0.84 (t, J= 7.35 Hz, 3H), 1.40-1.75 (m, 6H), 2.32 (s, 3H), 2.57 (s, 3H), 2.80-2.92 (m, 3H), 3.01 (d, J= 16.20 Hz, 1H), 4.31 (s, 2H), 6.13 (d, J= 8.67 Hz, 1H), 6.92-6.98 (m, 3H), 7.19 (d, J= 2.26 Hz, 1H), 7.26 (d, J= 7.91 Hz, 2H). LCMS (12 minute method) $[M+H]^+$ = 371/373 @Rt 5.75 min (100%).

**Example 12: 6-Chloro-1-(4-chloro-phenyl)-3-ethyl-3-(3-methylamino-propyl)-3,4-dihydro-*1H*-quinolin-2-one (13g)**

This was prepared from **(1c)** using the same synthetic sequence described in method B to give 222 mg of the racemate, which was purified by preparative LCMS. $^1$H NMR (300 MHz, $CDCl_3$) (racemate) δ ppm 0.84 (t, J= 7.44 Hz, 3H), 1.40-1.70 (m, 6H), 2.35 (br, 4H), 2.49-2.56 (m, 2H), 2.80 (d, J= 16.01 Hz, 1H), 2.90 (d, J= 16.01 Hz, 1H), 6.14 (d, J= 8.67 Hz, 1H), 6.93 (dd, J= 8.67, 2.26 Hz, 1H), 7.04 (ddd, J= 9.04, 2.83, 2.45 Hz, 2H), 7.09 (d, J= 2.26 Hz, 1H), 7.36-7.43 (m, 2H). LCMS (12 minute method) $[M+H]^+$ = 391/393 @Rt 5.67 min (92%).

**Method C**

**Preparation of intermediates**

**1-(4-Methoxy-benzyl)-3,4-dihydro-*1H*-quinolin-2-one (14)**

A 5 litre flange-neck flask equipped with an air stirrer and paddle, thermometer, nitrogen bubbler and pressure equalising dropping funnel was charged with sodium hydride (25.5g, 60% oil dispersion, 0.637 mol) and 40-60 pet. ether (100 ml). The mixture was stirred briefly and then allowed to settle under nitrogen. After decanting the supernatant liquid, the vessel was charged with dimethylformamide (2 litres). The well stirred suspension was cooled to 7-8°C using an external ice-bath. Then a soln of 3,4-dihydro-1H-quinolin-2-one **(1a)** (73.6g, 0.5 mole) in anhydrous dimethylformamide

(500 ml) was added dropwise over 25 min. The mixture was stirred at 7-8°C for 30 min. then 4-methoxybenzyl chloride (102 g, 0.65 mole, 1.3 eq.) was added over 10 min. The reaction mixture was left to stir for 2 h. at <10°C then allowed to warm-up to room temperature and stirred overnight. The stirred reaction mixture was quenched with ice/water (2.5 litres) and cooled to 15 °C using an external ice-bath. The white solid was isolated by filtration and washed with water. After drying in vacuo at 40°C overnight the product was obtained (113.4g, 85%).

**1-(4-Methoxy-benzyl)-3-methyl-3,4-dihydro-*1H*-quinolin-2-one (15)**

To a soln of **(14)** (20 g, 75 mmol) in anhydrous THF (400 mL) at -78°C under nitrogen was added LiHMDS (78.6 mL, 1M soln in hexanes, 78.6 mmol) dropwise over 10 min. The reaction mixture was left at -78°C for 30 min and then a solution of methyl iodide (5.13 mL, 83 mmol) in THF (5 mL) was added dropwise. The reaction mixture was warmed slowly to rt, quenched with water (50 mL) and extracted with ethyl acetate (400 mL). The organic layer was separated, dried over $MgSO_4$ and concentrated to give the product as a yellow solid (21 g, 100%) that was used directly in the next step.

**3-Allyl-1-(4-methoxy-benzyl)-3-methyl-3,4-dihydro-1*H*-quinolin-2-one (16b)**

To a soln of **(15)** (20.5 g, 73 mmol) in anhydrous THF (400 mL) at -78°C under nitrogen was added LiHMDS (80 mL, 1M soln in hexanes, 80 mmol) dropwise over 10 min. The reaction mixture was left at -78°C for 30 min and then a solution of allyl bromide (7.6 mL, 87 mmol) in THF (5 mL) was added dropwise. The reaction mixture was warmed slowly to rt, quenched with water (100 mL) and extracted with ethyl acetate (400 mL). The organic layer was separated, dried over $MgSO_4$ and concentrated to give the product as an orange oil (23.9 g, 100%) that was used directly in the next step.

**3-(3-Hydroxy-propyl)-1-(4-methoxy-benzyl)-3-methyl-3,4,4a,8a-tetrahydro-*1H*-quinolin-2-one (17b)**

To a soln of **(16b)** (23.9 g, 74 mmol) in anhydrous THF (400 mL) at 0°C under nitrogen was added 9-BBN (370 mL, 0.5M soln in THF, 185 mmol, 2.5 eq.) dropwise over 10 min. The reaction mixture was warmed to rt and left to stir overnight. The resultant yellow soln was cooled to 0°C and then quenched carefully with ethanol (95 mL), followed by aq. NaOH (60 mL, 3N soln). Finally, aq. $H_2O_2$ (60 mL, 37% soln) was added dropwise maintaining the internal reaction mixture temp between 5 and 10 °C. The reaction mixture was warmed to rt and then refluxed for 90 min. The reaction mixture was cooled to rt, poured into ethyl acetate and water and extracted. The organic layer was separated, dried over $MgSO_4$ and concentrated. The crude product was purified using automated chromatography (silica) (0 to 80% ethyl acetate\cyclohexane gradient) to provide the product as a clear oil (21.3 g, 84%).

**1-(4-Methoxy-benzyl)-3-met6yl-3-(3-methylamino-propyl)-3,4,4a,8a-tetrahydro-*1H*-quinolin-2-one (18b)**

To a soln of (**17b**) (18 g, 53 mmol) and triethylamine (11.1 mL, 79 mmol) in anhydrous THF (450 mL) at 0°C under nitrogen was added dropwise a soln of methanesulfonyl chloride (4.52 mL, 58 mmol) in THF (50 mL). The reaction mixture was warmed to rt and stirred for 3 h. The reaction mixture was poured into ethyl acetate and water and extracted. The organic layer was separated, dried over $MgSO_4$ and concentrated. The crude mesylate (22 g, 99%) was dissolved in ethanol (500 mL) and aqueous 40% methylamine (200 mL) and heated at 65°C under nitrogen for 2 h. The reaction mixture was cooled, concentrated and then extracted with ethyl acetate (300 mL). The organic layer was washed with water, brine, dried over $MgSO_4$ and concentrated to give the crude product (17.8 g, 96%).

**Methyl-[3-(3-methyl-2-oxo-1,2,3,4,4a,8a-hexahydro-quinolin-3-yl)-propyl]-carbamic acid *tert*-butyl ester (19b)**

A mixture of (**18b**) (17.8 g, 50.5 mmol) and anisole (5.5 mL, 50.5 mmol) in trifluoroacetic acid (250 mL) was heated at 65°C under nitrogen for 2 h. The reaction mixture was concentrated under vacuo and the residue was dissolved in methanol (10 mL). The methanol soln was applied to an SCX-2 column (300 g, prewashed with methanol) and the column washed with methanol (approx 1 litre) until the soln became colourless. The product was eluted with 2N $NH_3$ in methanol (500 mL) and the basic soln was concentrated to provide 3-Methyl-3-(3-methylaminopropyl)-3,4-dihydro-1H-quinolin-2-one (9 g, 77%). To a soln of this amine (8.6 g, 37 mmol) in anhydrous THF (350 mL) at 0°C was added a soln of di-*tert*-butyl dicarbonate (8.34 g, 97%, 50.5 mmol) in THF (20 mL) dropwise. The reaction mixture was warmed to rt and stirred for 3 h. The reaction mixture was poured into ethyl acetate (400 mL) and water (200 mL) and extracted. The organic layer was separated, dried over $MgSO_4$ and concentrated to give the product as a yellow solid (12.26 g, 100%). This material was used without further purification.

**Methyl-[3-(2-oxo-1,2,3,4-tetrahydro-quinolin-3-yl)-propyl]-carbamic acid tert-butyl ester (19a)**

This was prepared from **(14)** using the same synthetic sequence described in method C.

**[3-(6-Chloro-1,2,3,4-tetrahydro-quinolin-3-yl)-propyl]-methyl-carbamic acid *tert-butyl* ester (20a)**

To a soln of **(19a)** (2.75 g, 8.6 mmol) in anhydrous DMF (25 mL) at 0°C was added dropwise a soln of N-chlorosuccinimide (1.17 g, 8.7 mmol) in anhydrous DMF (3 mL). The reaction mixture was warmed to rt, stirred overnight and then poured into ethyl acetate (100 mL) and water (50 mL) and extracted. The organic layer was separated, dried over $MgSO_4$ and concentrated to provide the product as a yellow oil 3 g, 98%) that was used without further purification.

<u>**Examples**</u>

**Example 13: 3-(3-Methylamino-propyl)-1-*p*-tolyl-3,4-dihydro-*1H*-quinolin-2-one (21a)**

A stirred mixture of (**19a**) (100 mg. 0.31 mmol), $K_2CO_3$ (92 mg, 0.66 mmol), *trans*-cyclohexane-1,2-diamine (8 μL, 0.06 mmol) and 4-bromotoluene (162 mg, 0.94 mmol) in 1,4-dioxane (0.5 mL) was heated under a nitrogen atmosphere at 125°C for 5 min to deoxygenate the reaction mixture. Copper (I) iodide (12 mg, 0.06 mmol) was added in one portion and the reaction mixture was refluxed overnight at 125°C. After cooling to rt, the reaction mixture was poured into ethyl acetate (100 mL) and extracted with water. The organic layer was separated, dried over $MgSO_4$ and concentrated. The crude product was purified using automated chromatography (silica) (0 to 80% ethyl acetate\cyclohexane gradient) to provide the Boc protected product (70 mg, 54%). To a soln of this material (70 mg, 0.17 mol) in DCM (2 mL), was added trifluoroacetic acid (197 μL, 2.55 mmol, 15 eq.). The reaction mixture was left to stir at room temperature for 90 min, concentrated under vacuo poured into ethyl acetate (50 mL) and aq. $NaHCO_3$ (20 mL) and extracted. The organic layer was separated, dried over $MgSO_4$, concentrated and the crude product was purified by SCX-2 to provide the racemate (40 mg, 75%). The racemate was separated into its individual enantiomers using chiral HPLC. $^1$H NMR (300 MHz, $CDCl_3$) (racemate) δ 1.49-1.77 (m, 3H), 1.86-1.96 (m, 1H), 2.34 (bs, 1H), 2.40 (s, 3H), 2.43 (s, 3H), 2.61-2.66 (t, J= 6.88 Hz, 2H), 2.68-2.78 (m, 1H), 2.83-2.90 (m, 1H), 3.09-3.17 (m, 1H), 6.36 (dd, J= 7.7 Hz, 1.0 Hz, 1H), 6.94-7.03 (m, 2H), 7.08 (d, J= 8.2 Hz, 2H), 7.13-7.17 (m, 1H), 7.29 (d, J= 8.1 Hz, 2H); $^1$H NMR (300 MHz, MeOD-d4) (isomer, D-tartrate salt) δ 1.64 (bs, 1H), 1.89 (bs, 3H), 2.41(s, 3H), 2.70 (s, 3H), 2.75-2.87 (m, 1H), 2.91-3.06 (m, 3H), 3.20 (dd, J= 5.9, 15.26 Hz, 1H), 4.45 (s, 2H), 6.32-6.35 (m, 1H), 7.00-7.12 (m, 4H), 7.28-7.30 (m, 1H), 7.37 (d, J= 8.1 Hz, 2H). LCMS (12 minute method) $[M+H]^+$ = 309 @ Rt 4.7 min (100%).

**Example 14: 6-Chloro-3-(3-methylamino-propyl)-1-*p*-tolyl-3,4-dihydro-*1H*-quinolin-2-one (21n)**

This was prepared from **(20a)** (132 mg, 0.29 mmol) using the same methods described for **(21a)** to provide the racemate (86 mg). $^1$H NMR (300 MHz, $CDCl_3$) (racemate & isomer) δ 1.50-1.57 (m, 1H), 1.62-1.90 (m, 3H), 2.34 (s, 3H), 2.41 (s, 3H), 2.63-2.82 (m, 5H), 3.00-3.07 (m, 1H), 6.22 (d, J= 8.6 Hz, 1H), 6.92 (dd, J= 2.45, 8.66 Hz, 1H), 6.99 (d, J= 8.1 Hz, 2H), 7.11 (d, J= 2.25 Hz, 1H), 7.23 (d, J= 8.1 Hz, 2H). LCMS (12 minute method) $[M+H]^+$ = 343/345 @ Rt 5.2 min (96%).

**Example 15: 1-(3-Fluorophenyl)-3-(3-methylamino-propyl)-3,4-dihydro-*1H*-quinolin-2-one (21 b)**

This was prepared from **(19a)** (200 mg, 0.63 mmol) using the same two-step procedure described for **(21a)** to provide the racemate (83 mg). $^1$H NMR (300 MHz, $CDCl_3$) (racemate) δ 1.60-1.70 (m, 1H), 1.92 (br, 3H), 2.64 (bs, 3H), 2.72-2.74 (m, 1H), 2.86-3.09 (m, 4H), 6.35 (dd, J= 7.72, 1.510 Hz, 1H), 6.94-7.23 (m, 6H), 7.43-7.51 (m, 1H). LCMS (12 minute method) $[M+H]^+$ = 313 @ Rt 4.4 min (100%).

**Example 16: 1-(4-Chlorophenyl)-3-(3-methylamino-propyl)-3,4-dihydro-*1H*-quinolin-2-one (21c)**

This was prepared from **(19a)** (122 mg, 0.38 mmol) using the same two-step procedure described for **(21a)** to provide the crude product, which was purified by SCX-2 to give the racemate (70 mg). $^1$H NMR (300 MHz, $CDCl_3$) (racemate) δ 1.49-1.73 (m, 3H), 1.89 (m, 2H), 2.43 (s, 3H), 2.62 (t, J= 6.79, 7.15 Hz, 2H), 2.68-2.78 (m, 1H), 2.83-2.93 (m, 1H), 3.14 (dd, J= 15.43, 5.37 Hz, 1H), 6.34 (dd, J= 7.73, 1.14 Hz, 1H), 6.96-7.09 (m, 2H), 7.14-7.21 (m, 3H), 7.45-7.48 (m, 2H). LCMS (12 minute method) $[M+H]^+$ = 329/331 @ Rt 5.1 min (90%).

**Example 17: 1-(3,4-Dichlorophenyl)-3-(3-methylamino-propyl)-3,4-dihydro-*1H*-quinolin-2-one (21d)**

This was prepared from **(19a)** (150 mg, 0.47 mmol) using the same two-step procedure described for **(21a)** to provide the crude product, which was purified by SCX-2 to give the racemate (111 mg). $^1$H NMR (300 MHz, $CDCl_3$) (racemate) δ 1.49-1.75 (m, 3H), 1.83 (bs, 1H), 1.85-1.97 (m, 1H), 2.43 (s, 3H), 2.63 (t, J= 13.56, 6.59 Hz, 2H), 2.68-2.77 (m, 1H), 2.83-2.94 (m, 1H), 3.13 (dd, J= 15.45, 5.28 Hz, 1H), 6.36 (dd, J= 7.73, 0.93 Hz, 1H), 6.99-7.11 (m, 3H), 7.20-7.21 (m, 1H), 7.35 (d, J= 2.26 Hz, 1H), 7.57 (d, J= 8.48 Hz, 1H). LCMS (12 minute method) $[M+H]^+$ = 363/365 @Rt 5.4 min (92%).

**Example 18: 1-(3-Chlorophenyl)-3-(3-methylamino-propyl)-3,4-dihydro-*1H*-quinolin-2-one (21e)**

This was prepared from **(19a)** (200 mg, 0.63 mmol) using the same two-step procedure described for **(21a)** to provide the crude product, which was purified by SCX-2 to give the racemate (138 mg). $^1$H NMR (300 MHz, $CDCl_3$) (racemate) δ 1.50-1.77 (m, 3H), 1.89-1.96 (m, 2H), 2.44 (s, 3H), 2.64 (t, J= 6.89 Hz, 2H), 2.69-2.78 (m, 1H), 2.84-2.93 (m, 1H,), 3.10-3.17 (m, 1H), 6.33-6.36 (m, 1H), 6.97-7.10 (m, 2H), 7.11-7.15 (m, 1H), 7.21-7.24 (m, 2H), 7.37-7.47 (m, 2H). LCMS (12 minute method) $[M+H]^+$ = 329/331 @ Rt 5.01 min (90%).

**Example 19: 1-(4-Fluorophenyl)-3-(3-methylamino-propyl)-3,4-dihydro-*1H*-quinolin-2-one (21f)**

This was prepared from **(19a)** (200 mg, 0.63 mmol) using the same two-step procedure described for **(21a)** to provide the crude product, which was purified by SCX-2 to give the racemate (48 mg). $^1$H NMR (300 MHz, $CDCl_3$) (racemate) δ 1.26-1.28 (m, 1H), 1.92 (m, 2H), 2.63 (bs, 1H), 2.72 (m, 1H), 2.85-3.08 (m, 2H), 3.48-3.51 (m, 5H), 6.32-6.34 (d, J= 7.91 Hz, 1H), 7.01-7.70 (m, 2H), 7.16-7.19 (d, J= 7.16 Hz, 5H), 9.46 (bs, 1H). LCMS (12 minute method) $[M+H]^+$ = 313 @ Rt 4.5 min (100%).

**Example 20: 1-(4-Ethylphenyl)-3-(3-methylamino-propyl)-3,4-dihydro-*1H* quinolin-2-one (21g)**

This was prepared from **(19a)** (148 mg, 0.46 mmol) using the same two-step procedure described for **(21a)** to provide the racemate (61 mg). $^1$H NMR (300 MHz, $CDCl_3$) (racemate) δ 1.25-1.30 (m, 1H),1.52-1.67(m, 1H), 1.69-1.80 (m, 2H), 1.87-1.98 (m, 1H), 2.46 (s, 3H), 2.67-2.92 (m, 9H), 3.11-3.16 (m, 1H), 6.34-6.37 (m, 1H), 6.94-7.06 (m, 2H), 7.09-7.11 (d, J= 8.1 Hz, 2H), 7.17-7.20 (d, J= 7.35 Hz, 1H), 7.30-7.33 (d, J= 8.28 Hz, 2H). LCMS (12 minute method) $[M+H]^+$ = 323 @ Rt 5.4 min (98%).

**Example 21: 3-Methyl-3-(3-methylamino-propyl)-1-p-tolyl-3,4-dihydro-1H-quinolin-2-one (21h)**

This was prepared from (**19b**) (806 mg, 2.89 mmol) using the same methods described for **(21a)** to provide the racemate. The racemate was separated into its individual enantiomers using chiral HPLC. $^1$H NMR (300 MHz, $CDCl_3$) (racemate & isomer) δ 1.24 (s, 3H), 1.60-1.65 (m, 4H), 2.40 (s, 3H), 2.43 (s, 3H), 2.60-2.65 (m, 2H), 2.87 (d, J= 15.73 Hz, 1H), 2.98 (d, J= 15.73 Hz, 1H), 3.46 (br, 1H), 6.30 (dd, J= 7.91, 1.13 Hz, 1H), 6.90-7.05 (m, 2H), 7.05 (d, J= 8.29 Hz, 2H), 7.10-7.20 (m, 1H), 7.29 (d, J= 7.91 Hz, 2H). LCMS (12 minute method) [M+H] = 323 @Rt 5.06 min (100%).

**Example 22: 1-(4-Chlorophenyl)-3-methyl-3-(3-methylamino-propyl)-3,4-dihydro-*1H*-quinolin-2-one (21i)**

This was prepared from (**19b**) (100 mg, 0.30 mmol) using the same methods described for **(21a)** to provide the racemate (97 mg). 1H NMR (300 MHz, $CDCl_3$) (racemate) δ ppm 1.25 (s, 3H), 1.55-1.65 (m, 4H), 2.41 (s, 3H), 2.58 (m, 2H), 2.89 (d, J= 15.82 Hz, 1H), 2.98 (d, J= 15.82 Hz, 1H), 3.12 (br, 1H), 6.29 (dd, J= 7.91, 0.94 Hz, 1H), 6.95-7.10 (m, 2H), 7.14 (d, J= 8.67 Hz, 2H), 7.15 (m, 1H), 7.45 (d, J= 8.67 Hz, 2H). LCMS (12 minute method) $[M+H]^+$ = 343/345 @Rt 5.09 min (100%).

**Example 23: 1-(3,4-Difluorophenyl)-3-methyl-3-(3-methylamino-propyl)-3,4-dihydro-1*H*-quinolin-2-one (21j)**

This was prepared from (**19b**) (100 mg, 0.30 mmol) using the same two-step procedure described for (**21a**) to provide the crude product, which was purified by SCX-2 to give the racemate (100 mg). $^1$H NMR (300 MHz, $CDCl_3$) (racemate) δ ppm 1.25 (s, 3H), 1.55-1.65 (m, 4H), 2.41 (s, 3H), 2.50-2.60 (m, 2H), 2.89 (d, J= 15.45 Hz, 1H), 2.90 (s, 1H), 2.98 (d, J= 15.45 Hz, 1H), 6.30 (dd, J= 7.91, 1.13 Hz, 1H), 6.90-7.10 (m, 4H), 7.18 (dd, J= 7.16, 1.32 Hz, 1H), 7.22-7.35 (m, 1H). LCMS (12 minute method) $[M+H]^+$ = 345 @Rt 4.85 min (97%).

**Example 24: 3-Methyl-3-(3-methylamino-propyl)-1-*m*-tolyl-3,4-dihydro-*1H*-quinolin-2-one (21k)**

This was prepared from (**19b**) (100 mg, 0.30 mmol) using the same two-step procedure described for **(21a)** to provide the crude product, which was purified by SCX-2 to give the racemate (90 mg). $^1$H NMR (300 MHz, $CDCl_3$) (racemate) δ ppm 1.26 (s, 3H), 1.50-1.70 (m, 4H), 1.75 (s, 1H), 2.38 (s, 3H), 2.39 (s, 3H), 2.50-2.60 (m, 2H), 2.89 (d, J= 15.64 Hz, 1H), 2.98 (d, J= 15.64 Hz, 1H), 6.30 (dd, J= 7.82, 1.04 Hz, 1H), 6.90-7.07 (m, 4H), 7.18 (dd, J= 13.66, 7.63 Hz, 2H), 7.37 (t, J= 7.63 Hz, 1H). LCMS (12 minute method) $[M+H]^+$ = 323 @Rt 5.09 min (98%).

**Example 25: 1-(3,5-Difluorophenyl)-3-methyl-3-(3-methylamino-propyl)-3,4-dihydro-1*H*-quinolin-2-one (211)**

This was prepared from (**19b**) (100 mg, 0.30 mmol) using the same two-step procedure described for **(21a)** to provide the crude product, which was purified by SCX-2 to give the racemate (95 mg). $^1$H NMR (300 MHz, $CDCl_3$) (racemate) δ ppm 1.26 (s, 3H), 1.50-1.65 (m, 4H), 2.40 (s, 3H), 2.50-2.60 (m, 2H), 2.82 (br, 1H), 2.89 (d, J= 15.82 Hz, 1H), 2.97 (d, J= 15.82 Hz, 1H), 6.34 (dd, J= 8.01, 1.04 Hz, 1H), 6.74-6.83 (m, 2H), 6.83-6.92 (m, 1H), 6.97-7.13 (m, 2H), 7.19 (dd, J= 7.06, 1.22 Hz, 1H). LCMS (12 minute method) $[M+H]^+$ = 345 @ Rt 4.87 min, (97%).

**Example 26: 6-Chloro-3-(3-methylamino-propyl)-1-phenyl-3,4-dihydro-1H-quinolin-2-one (21m)**

This was prepared from **(20a)** (285 mg, 0.8 mmol) using the same two-step procedure described for **(21a)** to provide the crude product, which was purified by preparative LCMS to give the racemate (62 mg). $^1$H NMR (300 MHz, $CDCl_3$) (racemate) δ 1.49-1.76 (m, 3H), 1.86-1.95 (m, 1H), 2.33 (bs, 1H), 2.44 (s, 3H), 2.61-2.95 (m, 4H), 3.09-3.16 (m, 1H), 6.24-6.27 (d, J= 8.67 Hz, 1H), 6.99 (dd, J= 8.67, 2.26 Hz, 1H), 7.17-7.19 (m, 3H), 7.39-7.44 (m, 1H), 7.47-7.52 (m, 2H). LCMS (12 minute method) $[M+H]^+$ = 329/331 @ Rt 5.04 min (93%).

**Example 27: 6-Chloro-1-(4-chlorophenyl)-3-(3-methylamino-propyl)-3,4-dihydro-*1H*-quinolin-2-one (21o)**

This was prepared from **(20a)** (160 mg, 0.45 mmol) using the same two-step procedure described for **(21 a)** to provide the crude product, which was purified by preparative LCMS to give the racemate (52 mg). $^1$H NMR (300 MHz, $CDCl_3$) (racemate) δ 1.57-1.67 (m, 1H), 1.73-1.75 (m, 2H), 1.87-1.9 (m, 1H), 2.47 (s, 2H), 2.64 (s, 1H), 2.68-2.73 (m, 2H), 2.81-2.89 (m, 1H), 3.07-3.13 (m, 3H), 6.27 (d, J= 8.48 Hz, 1H), 7.02 (d, J= 8.48 Hz, 1H), 7.14 (d, J= 8.29 Hz, 2H), 7.19 (s, 1H), 7.47 (d, J= 8.29 Hz, 2H). LCMS (12 minute method) $[M+H]^+$ = 363/365 @ Rt 5.4 min (72%).

**Example 28: 6-Chloro-3-methyl-3-(3-methylamino-propyl)-1-*p*-tolyl-3,4-dihydro-*1H*-quinolin-2-one (21p)**

This was prepared from (**20b**) (490 mg, 1.34 mmol) using the same methods described for **(21a)** to provide the racemate (470 mg). The racemate was separated into its individual enantiomers using chiral HPLC. $^1$H NMR (300 MHz, $CDCl_3$) (racemate) δ 1.25 (s, 3H), 1.50-1.65 (m, 4H), 2.39 (s, 3H), 2.40 (s, 3H), 2.50-2.60 (m, 3H), 2.86 (d, J= 16.01 Hz, 1H), 2.94 (d, J= 16.01 Hz, 1H), 6.24 (d, J= 8.67 Hz, 1H), 6.97 (dd, J= 8.76, 2.35 Hz, 1H), 7.03 (d, J= 8.10 Hz, 2H), 7.14 (d, J= 2.26 Hz, 1H), 7.29 (d, J= 7.91 Hz, 2H); $^1$H NMR (300 MHz, MeOD-d4) (isomer hemi-D-tartrate salt) δ 1.15 (s, 3H), 1.50-1.75 (m, 4H), 2.32 (s, 3H), 2.51 (s, 3H), 2.78 (br, 2H), 2.84 (d, J= 16.20 Hz, 1H), 2.98 (m, 1H), 3.15-3.25 (m, 2H), 4.22 (s, 1H), 6.14 (d, J= 8.85 Hz, 1H), 6.90-6.70 (m, 3H), 7.19 (d, J= 2.26 Hz, 1H), 7.25 (d, J= 7.91 Hz, 2H). LCMS (12 minute method) $[M+H]^+$ = 357/359 @Rt 5.43 min (100%).

**Example 29: 6-Chloro-1-(4-chlorophenyl)-3-methyl-3-(3-methylamino-propyl)-3,4-dihydro-*1H*-quinolin-2-one (21q)**

This was prepared from (**20b**) (490 mg, 1.34 mmol) using the same methods described for (**21a**) to provide the racemate (425 mg). $^1$H NMR (300 MHz, $CDCl_3$) (racemate) δ ppm 1.25 (s, 3H), 1.50-1.65 (m, 4H), 2.39 (s, 3H), 2.40 (br, 1H), 2.50-2.60 (m, 2H), 2.87 (d, J= 16.20 Hz, 1H), 2.95 (d, J= 16.20 Hz, 1H), 6.23 (d, J= 8.85 Hz, 1H), 7.00 (dd, J= 8.57, 2.35 Hz, 1H), 7.05-7.20 (m, 3H), 7.40-7.50 (m, 2H). LCMS (12 minute method) $[M+H]^+$ = 377/379 @Rt 5.26 min (94%).

**Example 30: 3-Methyl-3-(3-methylamino-propyl)-1-thiophen-2-yl-3,4-dihydro-1H-quinolin-2-one (22a)**

This was prepared from (**19b**) (200 mg, 0.60 mmol) using the same two-step procedure described for (**21a**) to provide the crude product, which was purified by SCX-2 to give the racemate (125 mg). $^1$H NMR (300 MHz, $CDCl_3$) (racemate) δ ppm 1.25 (s, 3H), 1.50-1.65 (m, 4H), 2.39 (s, 3H), 2.50-2.60 (br, 2H), 2.88 (d, J= 16.20 Hz, 1H), 2.97 (d, J= 16.20 Hz, 1H), 3.17 (br, 1H), 6.58 (dd, J= 8.01, 0.85 Hz, 1H), 6.89 (dd, J= 3.58, 1.32 Hz, 1H), 6.95-7.15 (m, 3H), 7.16

(d, J= 7.16 Hz, 1H), 7.32 (dd, J= 5.65, 1.32 Hz, 1H). LCMS (12 minute method) $[M+H]^+$ = 315 @Rt 4.35 min (98%).

**Example 31: 3-Methyl-3-(3-methylamino-propyl)-1-thiophen-3-yl-3,4-dihydro-1H-quinolin-2-one (22b)**

This was prepared from (**19b**) (200 mg, 0.60 mmol) using the same two-step procedure described for (**21a**) to provide the crude product, which was purified by SCX-2-2 to give the racemate (128 mg). $^1H$ NMR (300 MHz, $CDCl_3$) δ 1.24 (s, 3H), 1.50-1.65 (m, 4H), 2.40 (s, 3H), 2.50-2.60 (m, 2H), 2.87 (d, J= 15.82 Hz, 1H), 2.96 (d, J= 15.82 Hz, 1H), 3.07 (br, 1H), 6.45 (dd, J= 8.10, 0.94 Hz, 1H), 6.92 (dd, J= 5.09, 1.32 Hz, 1H), 6.98 (td, J= 7.35, 1.13 Hz, 1H), 7.07 (td, J= 7.77, 1.60 Hz, 1H), 7.16 (d, J= 7.35 Hz, 1H), 7.22 (dd, J= 3.20, 1.32 Hz, 1H), 7.41 (dd, J= 5.09, 3.20 Hz, 1H). LCMS (12 minute method) $[M+H]^+$ = 315 @Rt 4.29 min (100%).

**Method D**

**Preparation of intermediates**

**{3-[1-(4-Methoxy-benzyl)-3-methyl-2-oxo-6-phenyl-1,2,3,4-tetrahydroquinolin-3-yl]-propyl}-methyl-carbamic acid *tert*-butyl ester (23)**

**Step (i)**

Sodium hydride (340 mg, 60% dispersion in mineral oil, 8.55 mmol, 1.3 eq.) was added portionwise to a soln of **(20c)** (2.7 g. 6.57 mmol) in DMF (40 mL) at 0°C. The reaction mixture was left for 30 min at this temperature and then 4-methoxybenzyl chloride (1.16 mL, 8.55 mmol, 1.3 eq.) in DMF (1 mL) was added dropwise over 10 min. The reaction mixture was warmed to rt slowly and after 1 h was poured into ethyl acetate (200 mL) and extracted with water (3 x 50 mL). The organic layer was separated, dried over $MgSO_4$ and concentrated under vacuo. The crude product was purified using automated chromatography (silica) (0 to 80% ethyl acetate\cyclohexane gradient) to provide the 4-methoxybenzyl protected 6-bromo precursor (2.2 g, 63%).

**Step (ii)**

The product from Step (i) (100 mg, 0.23 mmol), phenylboronic acid (85 mg, 0.70 mmol, 3 eq.), $K_2CO_3$ (138 mg, 1 mmol, 4.3 eq.) and $Pd(PPh_3)_4$ (11 mg, 0.009 mmol, 0.04 eq.) were suspended in ethanol (1 mL) and water (0.6 mL). The reaction mixture was heated at 80°C overnight, cooled to rt and filtered through celite. The filtrate was poured into ethyl acetate (100 mL) and water (50 mL) and extracted. The organic layer was separated, dried over $MgSO_4$ and concentrated to provide the product (23) (120 mg, 98%) that was used without further purification.

**Methyl-[3-(3-methyl-2-oxo-6-phenyl-1,2,3,4-tetrahydro-quinolin-3-yl)-propyl]-carbamic acid *tert*-butyl ester**

**Step (iii) & (iv)**

A mixture of **(23)** (120 mg, 0.23 mmol) and anisole (25 μL, 0.23 mmol) in trifluoroacetic acid (2.3 mL) was heated at 65°C under nitrogen for 4 h. The reaction mixture was concentrated under vacuo and the residue was dissolved in methanol (2 mL). The methanol soln was applied to an SCX-2 column (5g) and the column washed with methanol (50 mL). The product was eluted with 2N $Et_3N$ in methanol (50 mL) and the basic soln was concentrated to provide 3-Methyl-3-(3-methylamino-propyl)-6-phenyl-3,4-dihydro-*1H*-quinolin-2-one (72 mg, 100%). To a soln of this amine (72 mg, 0.23 mmol) in anhydrous THF (2 mL) at 0°C was added di-tert-butyl dicarbonate (53 mg, 97%, 0.24 mmol) in one portion. The reaction mixture was warmed to rt and stirred for 3 h. The reaction mixture was poured into ethyl acetate (25 mL) and water (10 mL) and extracted. The organic layer was separated, dried over $MgSO_4$ and concentrated to give the Boc protected precursor (95 mg, 100%). This material was used without further purification.

**Examples**

**Example 32: 3-Methyl-3-(3-methylamino-propyl)-6-phenyl-1-*p*-tolyl-3,4-dihydro-1H-quinolin-2-one (24)**

This was prepared from the above Boc protected precursor (95 mg, 0.23 mmol) using the same two-step procedure described for Method C **(19a** to **21a)** to provide the crude product, which was purified by SCX-2 to give the racemate (53 mg). $^1H$ NMR (300 MHz, $CDCl_3$) (racemate) δ 1.29 (s, 3H), 1.50-1.70 (m, 4H), 2.42 (s, 6H), 2.55-2.65 (m, 2H), 2.94 (d, J= 15.64 Hz, 1H), 3.04 (d, J= 15.64 Hz, 1H), 3.18 (br, 1H), 6.38 (d, J= 8.29 Hz, 1H), 7.09 (d, J= 8.10 Hz,

2H), 7.29 (m, 4H), 7.41 (m, 3H), 7.54 (m, 2H). LCMS (12 minute method) $[M+H]^+$ = 399 @Rt 6.06 min (100%).

The pharmacological profile of the present compounds may be demonstrated as follows.

Generation of stable cell-lines expressing the human dopamine, norepinephrine and serotonin transporters

Standard molecular cloning techniques are used to generate stable cell-lines expressing the human dopamine, norepinephrine and serotonin transporters. The polymerase chain reaction (PCR) is used in order to isolate and amplify each of the three full-length cDNAs from an appropriate cDNA library. Primers for PCR are designed using the following published sequence data:

Human dopamine transporter: GenBank M95167. Reference: Vandenbergh DJ, Persico AM and Uhl GR. A human dopamine transporter cDNA predicts reduced glycosylation, displays a novel repetitive element and provides racially-dimorphic TaqI RFLPs. Molecular Brain Research (1992) volume 15, pages 161-166.

Human norepinephrine transporter: GenBank M65105. Reference: Pacholczyk T, Blakely, RD and Amara SG. Expression cloning of a cocaine- and antidepressant-sensitive human noradrenaline transporter. Nature (1991) volume 350, pages 350-354.

Human serotonin transporter: GenBank L05568. Reference: Ramamoorthy S, Bauman AL, Moore KR, Han H, Yang-Feng T, Chang AS, Ganapathy V and Blakely RD. Antidepressant- and cocaine-sensitive human serotonin transporter: Molecular cloning, expression, and chromosomal localization. Proceedings of the National Academy of Sciences of the USA (1993) volume 90, pages 2542-2546.

The PCR products are cloned into a mammalian expression vector (eg pcDNA3.1 (Invitrogen)) using standard ligation techniques. The constructs are then used to stably transfect HEK293 cells using a commercially available lipofection reagent (Lipofectamine™ - Invitrogen) following the manufacture's protocol.

Norepinephrine Binding Assay

The ability of compounds to compete with $[^3H]$-Nisoxetine for its binding sites on cloned human norepinephrine membranes is used as a measure of its ability to block norepinephrine uptake via its specific transporter.

Membrane Preparation:

Cell pastes from large scale production of HEK-293 cells expressing cloned human noradrenaline transporters are homogenised in 4 volumes 50mM Tris.HCl containing 300mM NaCl and 5mM KCl, pH 7.4. The homogenate is centrifuged twice (40,000g, 10min, 4°C) with pellet re-suspension in 4 volumes Tris.HCl buffer after the first spin and 8 volumes after the second spin. The suspended homogenate is centrifuged (100g, 10min, 4°C) and the supernatant kept and re-centrifuged (40,000g, 20min, 4°C). The pellet is resuspended in Tris.HCl buffer containing the above reagents along with 10%w/v sucrose and 0.1 mM phenylmethylsulfonyl fluoride (PMSF). The membrane preparation is stored in aliquots (1ml) at -80°C until required. The protein concentration of the membrane preparation is determined using a bicinchoninic acid (BCA) protein assay reagent kit (available from Pierce).

$[^3H]$-Nisoxetine Binding Assay:

Each well of a 96well microtitre plate is set up to contain the following:

| | |
|---|---|
| 50μl | 2nM [N-methyl-$^3$H]-Nisoxetine hydrochloride (70-87Ci/mmol, from NEN Life Science Products) |
| 75μl | Assay buffer (50mM Tris.HCl pH 7.4 containing 300mM NaCl and 5mM KCl) |
| 25μl | Test compound, assay buffer (total binding) or 10μM Desipramine HCl (non-specific binding) |
| 50μl | Wheatgerm agglutinin coated poly(vinyltoluene) (WGA PVT) SPA Beads (Amersham Biosciences RPNQ0001) (10mg/ml) |
| 50μl | Membrane (0.2mg protein per ml.) |

The microtitre plates are incubated at room temperature for 10 hours prior to reading in a Trilux scintillation counter. The results are analysed using an automatic spline fitting programme (Multicalc, Packard, Milton Keynes, UK) to provide Ki values for each of the test compounds.

Serotonin Binding Assay

The ability of a test compound to compete with $[^3H]$-citalopram from its binding sites on cloned human serotonin

membranes is used as a measure of its ability to block serotonin uptake via its specific transporter (Ramamoorthy, S., Giovanetti, E., Qian, Y., Blakely, R., (1998) J. Biol. Chem. 273,2458).

Membrane Preparation:

The preparation of membrane is essentially similar to that for the norepinephrine transporter containing membrane described above. The membrane preparation is stored in aliquots (1ml) at -70°C until required. The protein concentration of the membrane preparation is determined using BCA protein assay reagent kit.

[$^3$H]-Citalopram Binding Assay:

Each well of a 96well microtitre plate is set up to contain the following:

| | |
|---|---|
| 50μl | 2nM [$^3$H]-Citalopram (60-86Ci/mmol, Amersham Biosciences) |
| 75μl | Assay buffer (50mM Tris.HCl pH 7.4 containing 150mM NaCl and 5mM KCl) |
| 25μl | Diluted compound, assay buffer (total binding) or 100μM Fluoxetine (non-specific binding) |
| 50μl | WGA PVT SPA Beads (40mg/ml) |
| 50μl | Membrane preparation (0.4mg protein per ml) |

The microtitre plates are incubated at room temperature for 10 hours prior to reading in a Trilux scintillation counter. The results are analysed using an automatic spline fitting programme (Multicalc, Packard, Milton Keynes, UK) to provide Ki (nM) values for each of the test compounds.

Dopamine Binding Assay

The ability to compete with [$^3$H]-WIN35,428 for its binding sites on human cell membranes containing cloned human dopamine transporter is used as a measure of its ability to block dopamine uptake via its specific transporter (Ramamoorthy et al 1998 *supra*).

Membrane Preparation:

Is essentially the same as for membranes containing cloned human serotonin transporter as described above.

[$^3$H]-WIN35,428 Binding Assay:

Each well of a 96well microtitre plate is set up to contain the following:

| | |
|---|---|
| 50μl | 4nM [$^3$H]-WIN35,428428 (84-87Ci/mmol, from NEN Life Science Products) |
| 75μl | Assay buffer (50mM Tris.HCl pH 7.4 containing 150mM NaCl and 5mM KCl) |
| 25μl | Diluted compound, assay buffer (total binding) or 100μM Nomifensine (non-specific binding) |
| 50μl | WGA PVT SPA Beads (10mg/ml) |
| 50μl | Membrane preparation (0.2mg protein per ml.) |

The microtitre plates are incubated at room temperature for 120 minutes prior to reading in a Trilux scintillation counter. The results are analysed using an automatic spline fitting programme (Multicalc, Packard, Milton Keynes, UK) to provide Ki values for each of the test compounds.

Acid Stability

The acid stability of a compound according to the present invention was determined as a solution in buffer at 6 different pH values (HCl 0.1N, pH 2, pH 4, pH 6, pH 7, and pH 8) at 40°C over a time course of 72 hours. Samples were taken at the beginning of the study and after 3, 6 and 24 hours and analysed by capillary electrophoresis. The original sample used in this study contained 0.8% of the undesired epimer as internal standard. The samples taken at the different time points during the study did not show any significant change in the percentage of the undesired epimer. This confirms that the compound is chemically and configurationally stable under acidic conditions.

CYP2D6 Assays

Cytochrome P450 2D6 (CYP2D6) is a mammalian enzyme which is commonly associated with the metabolism of around 30% of pharmaceutical compounds. Moreover, this enzyme exhibits genetic polymorphism, resulting in the presence of both normal and poor metabolizers in the population. A low involvement of CYP2D6 in the metabolism of compounds (i.e. the compound being a poor substrate of CYP2D6) is desirable in order to reduce any variability from subject to subject in the pharmacokinetics of the compound. Also, compounds with a low inhibitor potential for CYP2D6 are desirable in order to avoid drug-drug interactions with co-administered drugs that are substrates of CYP2D6. Compounds may be tested both as substrates and as inhibitors of this enzyme by means of the following assays.

**CYP2D6 substrate assay**

**Principle:**

This assay determines the extent of the CYP2D6 enzyme involvement in the total oxidative metabolism of a compound in microsomes. Preferred compounds of the present invention exhibit less than 75% total metabolism via the CYP2D6 pathway.

For this in vitro assay, the extent of oxidative metabolism in human liver microsomes (HLM) is determined after a 30 minute incubation in the absence and presence of Quinidine, a specific chemical inhibitor of CYP2D6. The difference in the extent of metabolism in absence and presence of the inhibitor indicates the involvement of CYP2D6 in the metabolism of the compound.

**Materials and Methods:**

Human liver microsomes (mixture of 20 different donors, mixed gender) are acquired from Human Biologics (Scottsdale, AZ, USA). Quinidine and β-NADPH (β-Nicotinamide Adenine Dinucleotide Phosphate, reduced form, tetrasodium salt) are purchased from Sigma (St Louis, MO, USA). All the other reagents and solvents are of analytical grade. A stock solution of the new chemical entity (NCE) is prepared in a mixture of Acetonitrile/Water to reach a final concentration of acetonitrile in the incubation below 0.5%.

The microsomal incubation mixture (total volume 0.1 mL) contains the NCE (4 μM), β-NADPH (1mM), microsomal proteins (0.5 mg/mL), and Quinidine (0 or 2 μM) in 100 mM sodium phosphate buffer pH 7.4. The mixture is incubated for 30 minutes at 37 °C in a shaking waterbath. The reaction is terminated by the addition of acetonitrile (75 μL). The samples are vortexed and the denaturated proteins are removed by centrifugation. The amount of NCE in the supernatant is analyzed by liquid chromatography/mass spectrometry (LC/MS) after addition of an internal standard. A sample is also taken at the start of the incubation (t=0), and analysed similarly.

Analysis of the NCE is performed by liquid chromatography/mass spectrometry. Ten μL of diluted samples (20 fold dilution in the mobile phase) are injected onto a Spherisorb CN Column, 5 μM and 2.1 mm x 100 mm (Waters corp. Milford, MA, USA). The mobile phase consisting of a mixture of Solvent A/Solvent B, 30/70 (v/v) is pumped (Alliance 2795, Waters corp. Milford, MA, USA) through the column at a flow rate of 0.2 ml/minute. Solvent A and Solvent B are a mixture of ammonium formate $5.10^{-3}$ M pH 4.5/ methanol in the proportions 95/5 (v/v) and 10/90 (v/v), for solvent A and solvent B, respectively. The NCE and the internal standard are quantified by monitoring their molecular ion using a mass spectrometer ZMD or ZQ (Waters-Micromass corp, Manchester, UK) operated in a positive electrospray ionisation.

The extent of CYP2D6 involvement (% of CYP2D6 involvement) is calculated comparing the extent of metabolism in absence and in presence of quinidine in the incubation.

The extent of metabolism without inhibitor (%) is calculated as follows:

$$\frac{(\text{NCE response in samples without inhibitor})_{\text{time }0} - (\text{NCE response in samples without inhibitor})_{\text{time }30}}{(\text{NCE response in samples without inhibitor})_{\text{time }0}} \times 100$$

The extent of metabolism with inhibitor (%) is calculated as follows:

$$\frac{(\text{NCE response in samples without inhibitor})\text{time 0} - (\text{NCE response in samples with inhibitor})\text{time 30}}{(\text{NCE response in samples without inhibitor})\text{time 0}} \times 100$$

where the NCE response is the area of the NCE divided by the area of the internal standard in the LC/MS analysis chromatogram, time0 and time30 correspond to the 0 and 30 minutes incubation time.

The % of CYP2D6 involvement is calculated as follows:

$$\frac{(\%\ \text{extent of metabolism without inhibitor}) - (\%\ \text{extent of metabolism with inhibitor})}{\%\ \text{extent of metabolism without inhibitor}} \times 100$$

CYP2D6 inhibitor assay

**Principle:**

The CYP2D6 inhibitor assay evaluates the potential for a compound to inhibit CYP2D6. This is performed by the measurement of the inhibition of the bufuralol 1'-hydroxylase activity by the compound compared to a control. The 1'-hydroxylation of bufuralol is a metabolic reaction specific to CYP2D6. Preferred compounds of the present invention exhibit an $IC_{50}$ higher than 6 μM for CYP2D6 activity, the $IC_{50}$ being the concentration of the compound that gives 50 % of inhibition of the CYP2D6 activity.

**Material and methods:**

Human liver microsomes (mixture of 20 different donors, mixed gender) are acquired from Human Biologics (Scottsdale, AZ). β-NADPH is purchased from Sigma (St Louis, MO). Bufuralol is purchased from Ultrafine (Manchester, UK). All the other reagents and solvents are of analytical grade.

Microsomal incubation mixture (total volume 0.1 mL) contains bufuralol 10 μM, β-NADPH (2 mM), microsomal proteins (0.5 mg/mL), and the new chemical entity (NCE) (0, 5, and 25 μM) in 100 mM sodium phosphate buffer pH 7.4. The mixture is incubated in a shaking waterbath at 37 °C for 5 minutes. The reaction is terminated by the addition of methanol (75 μL). The samples are vortexed and the denaturated proteins are removed by centrifugation. The supernatant is analyzed by liquid chromatography connected to a fluorescence detector. The formation of the 1'-hydroxybufuralol is monitored in control samples (0 μM NCE) and in the samples incubated in presence of the NCE. The stock solution of NCE is prepared in a mixture of Acetonitrile/Water to reach a final concentration of acetonitrile in the incubation below 1.0%.

The determination of l'hydroxybufuralol in the samples is performed by liquid chromatograhy with fluorimetric detection as described below. Twenty five μL samples are injected onto a Chromolith Performance RP-18e column (100 mm x 4.6 mm) (Merck KGAa, Darmstadt, Germany). The mobile phase, consisting of a mixture of solvent A and solvent B whose the proportions changed according the following linear gradient, is pumped through the column at a flow rate of 1 ml/min:

| Time (minutes) | Solvent A (%) | Solvent B (%) |
|---|---|---|
| 0 | 65 | 35 |
| 2.0 | 65 | 35 |
| 2.5 | 0 | 100 |
| 5.5 | 0 | 100 |
| 6.0 | 65 | 35 |

Solvent A and Solvent B consist of a mixture of 0.02 M potassium dihydrogenophosphate buffer pH3/ methanol in the proportion 90/10 (v/v) for solvent A and 10/90 (v/v) for solvent B. The run time is 7.5 minutes. Formation of 1'-hydroxybufuralol is monitored by fluorimetric detection with extinction at λ 252 nm and emission at λ 302 nm.

The $IC_{50}$ of the NCE for CYP2D6 is calculated by the measurement of the percent of inhibition of the formation of the 1'-hydroxybufuralol in presence of the NCE compared to control samples (no NCE) at a known concentration of the NCE.

The percent of inhibition of the formation of the 1'-hydroxybufuralol is calculated as follows:

$$\frac{(\text{1'-hydroxybufuralol formed without inhibitor}) - (\text{1'-hydroxybufuralol formed with inhibitor})}{(\text{1'-hydroxybufuralol area formed without inhibitor})} \times 100$$

The $IC_{50}$ is calculated from the percent inhibition of the formation of the 1'-hydroxybufuralol as follows (assuming competitive inhibition):

$$\frac{\text{NCE Concentration} \times (100 - \text{Percent of inhibition})}{\text{Percent of inhibition}}$$

The $IC_{50}$ estimation is assumed valid if inhibition is between 20% and 80% (Moody GC, Griffin SJ, Mather AN, McGinnity DF, Riley RJ. 1999. Fully automated analysis of activities catalyzed by the major human liver cytochrome P450 (CYP) enzymes: assessment of human CYP inhibition potential. Xenobiotica, 29(1): 53-75).

## Claims

1. A compound of formula (I)

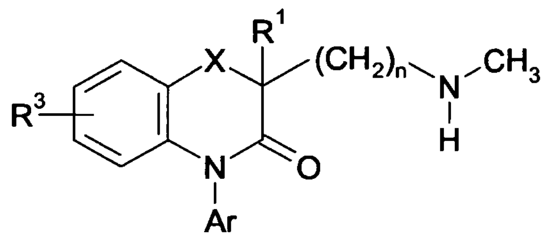

(I)

wherein
-X- is $-C(R^4R^5)-$, -O- or -S-;
n is 2 or 3;
$R^1$ is H or $C_1$-$C_4$ alkyl;
$R^3$ is H, halo, $C_1$-$C_4$ alkyl, O($C_1$-$C_4$ alkyl), nitrile, phenyl or phenyl substituted with 1 or 2 substituents selected from $C_1$-$C_4$ alkyl, O($C_1$-$C_4$ alkyl), S($C_1$-$C_4$ alkyl), halo, and phenyl optionally substituted with $C_1$-$C_4$ alkyl, O($C_1$-$C_4$ alkyl), S($C_1$-$C_4$ alkyl), or halo;
$R^4$ and $R^5$ are each independently selected from H or $C_1$-$C_4$ alkyl;
Ar- is selected from the group consisting of

(i) 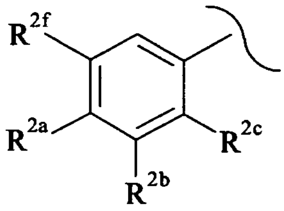 and (ii) 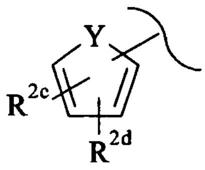

in which
$R^{2a}$ is H, halo, methyl or ethyl;
$R^{2b}$ is H, halo or methyl;
$R^{2c}$ is H, halo, methyl, trifluoromethyl, nitrile or methoxy;
$R^{2d}$ is H, halo, methyl or ethyl;
$R^{2c}$ is H, halo, methyl, trifluoromethyl, nitrile or methoxy;
$R^{2f}$ is H, or fluoro;
-Y- is -0-, -S- or -N($R^6$)-; and
$R^6$ is H or methyl;
or a pharmaceutically acceptable salt thereof.

**2.** A compound as claimed in claim 1, represented by the formula (Ia)

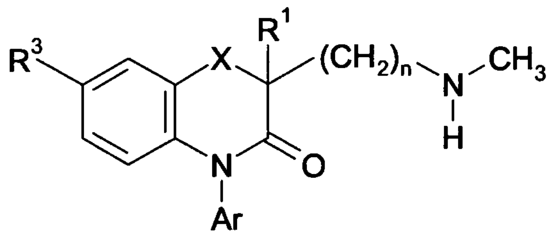

(Ia)

wherein -X-, n, $R^1$, $R^3$ and Ar have the values as defined for formula (I) in claim 1.

**3.** A compound as claimed in either claim 1 or 2, wherein -X- is -C($R^4R^5$)-.

**4.** A compound as claimed in any one of claims 1 to 3, wherein Ar is (i).

**5.** A compound as claimed in any one of claims 1 to 3, wherein Ar is (ii) and -Y- is -S-.

**6.** A compound as claimed in any one of claims 1 to 3, represented by the formula (II)

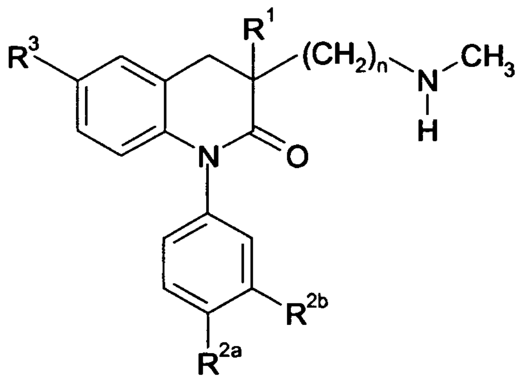

(II)

wherein n, $R^1$, $R^{2a}$ and $R^{2b}$ have the values as defined for formula (I) in claim 1 and $R^3$ is H, halo, phenyl or phenyl substituted with 1 or 2 substituents selected from $C_1$-$C_4$ alkyl, O($C_1$-$C_4$ alkyl), S($C_1$-$C_4$ alkyl), halo, and phenyl optionally substituted with $C_1$-$C_4$ alkyl, O($C_1$-$C_4$ alkyl), S($C_1$-$C_4$ alkyl), or halo.

**7.** A compound as claimed in any one of claims 1 to 6, wherein $R^3$ is H or halo.

**8.** A pharmaceutical composition comprising a compound as claimed in any one of claims 1 to 7 or a pharmaceutically acceptable salt thereof, together with a pharmaceutically acceptable diluent or carrier.

**9.** A compound as claimed in any one of claims 1 to 7 or a pharmaceutically acceptable salt thereof, for use in therapy.

**10.** Use of a compound as claimed in any one of claims 1 to 7 or a pharmaceutically acceptable salt thereof, for the manufacture of a medicament for treating a disorder selected from the group consisting of an attention-deficit disorder (ADD) due to general medical conditions, attention-deficit hyperactivity disorder (ADHD), conduct disorder, depression, oppositional defiant disorder, and cognitive disorders including mild cognitive impairment (MCI), dementia of the Alzheimers type (DAT), vascular dementia and cognitive impairment associated with schizophrenia (CIAS).

**Patentansprüche**

**1.** Verbindung der Formel (I)

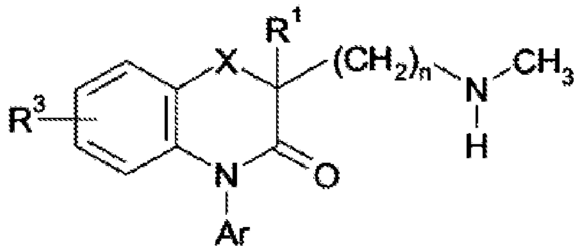

(I)

worin
-X- für -C($R^4R^5$)-, -O- oder -S- steht,
n für 2 oder 3 steht,

$R^1$ für H oder $C_1$-$C_4$-Alkyl steht,
$R^3$ für H, Halogen, $C_1$-$C_4$-Alkyl, O($C_1$-$C_4$-Alkyl), Nitril, Phenyl, oder Phenyl steht, das durch 1 oder 2 Substituenten substituiert ist, die ausgewählt sind aus $C_1$-$C_4$-Alkyl, O($C_1$-$C_4$-Alkyl), S($C_1$-$C_4$-Alkyl), Halogen und Phenyl, das optional substituiert ist durch $C_1$-$C_4$-Alkyl, O($C_1$-$C_4$-Alkyl), S($C_1$-$C_4$-Alkyl) oder Halogen,
$R^4$ und $R^5$ jeweils unabhängig ausgewählt sind aus H oder $C_1$-$C_4$-Alkyl,
Ar- aus der Gruppe ausgewählt ist, die besteht aus

(i) 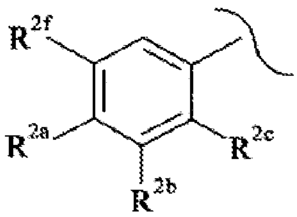 und (ii) 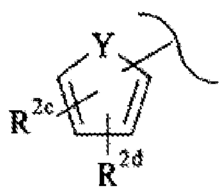

worin
$R^{2a}$ für H, Halogen, Methyl oder Ethyl steht,
$R^{2b}$ für H, Halogen oder Methyl steht,
$R^{2c}$ für H, Halogen, Methyl, Trifluormethyl, Nitril oder Methoxy steht,
$R^{2d}$ für H, Halogen, Methyl oder Ethyl steht,
$R^{2e}$ für H, Halogen, Methyl, Trifluormethyl, Nitril oder Methoxy steht,
$R^{2f}$ für H oder Fluor steht,
-Y- für -O-, -S- oder -N($R^6$)- steht und
$R^6$ für H oder Methyl steht,
oder ein pharmazeutisch akzeptables Salz hiervon.

**2.** Verbindung nach Anspruch 1 der Formel (Ia)

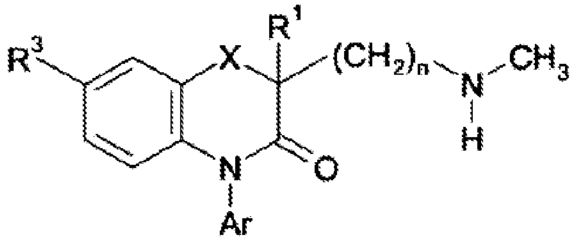

(Ia)

worin -X-, n, $R^1$, $R^3$ und Ar die im Anspruch 1 für die Formel (I) definierten Bedeutungen haben.

**3.** Verbindung nach Anspruch 1 oder 2, worin -X- für -C($R^4R^5$)- steht.

**4.** Verbindung nach einem der Ansprüche 1 bis 3, worin Ar für die Gruppe (i) steht.

**5.** Verbindung nach einem der Ansprüche 1 bis 3, worin Ar für die Gruppe (ii) steht und -Y- für -S- steht.

**6.** Verbindung nach einem der Ansprüche 1 bis 3 der Formel (II)

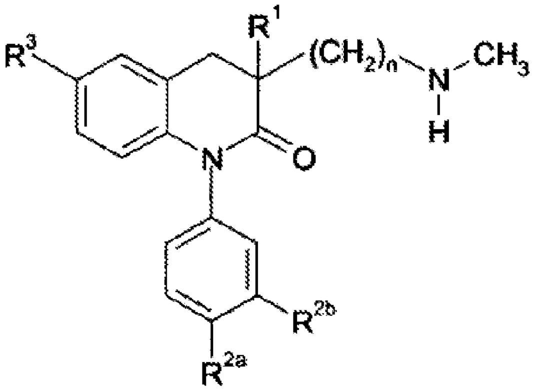

(II)

worin n, $R^1$, $R^{2a}$ und $R^{2b}$ die im Anspruch 1 für die Formel (I) definierten Bedeutungen haben und $R^3$ für H, Halogen, Phenyl oder Phenyl steht, das durch 1 oder 2 Substituenten substituiert ist, die ausgewählt sind aus $C_1$-$C_4$-Alkyl, O($C_1$-$C_4$-Alkyl), S($C_1$-$C_4$-Alkyl), Halogen und Phenyl, das optional substituiert ist durch $C_1$-$C_4$-Alkyl, O($C_1$-$C_4$-Alkyl), S($C_1$-$C_4$-Alkyl) oder Halogen.

**7.** Verbindung nach einem der Ansprüche 1 bis 6, worin $R^3$ für H oder Halogen steht.

**8.** Pharmazeutische Zusammensetzung, umfassend eine Verbindung nach einem der Ansprüche 1 bis 7 oder ein pharmazeutisch akzeptables Salz hiervon, zusammen mit einem pharmazeutisch akzeptablen Verdünnungsmittel oder Träger.

**9.** Verbindung nach einem der Ansprüche 1 bis 7 oder ein pharmazeutisch akzeptables Salz hiervon, zur Verwendung in der Therapie.

**10.** Verwendung einer Verbindung nach einem der Ansprüche 1 bis 7 oder eines pharmazeutisch akzeptablen Salzes hiervon zur Herstellung eines Arzneimittels für die Behandlung einer Störung, die aus der Gruppe ausgewählt ist, die besteht aus einer Aufmerksamkeitsdefizitstörung (ADD) infolge allgemeiner medizinischer Zustände, Aufmerksamkeitsdefizithyperaktivitätsstörung (ADHD), Konduktstörung, Depression, oppositionalen Trotzstörung und Kognitionsstörungen unter Einschluss einer schwachen Kognitionsbeeinträchtigung (MCI), Demenz des Alzheimertyps (DAT), Vaskulardemenz und Kognitionsbeeinträchtigung in Assoziation mit Schizophrenie (CIAS).

## Revendications

**1.** Composé de formule I,

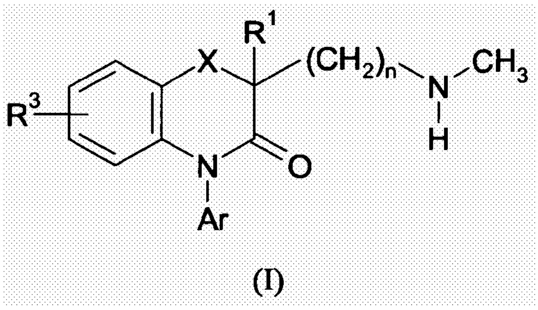

(I)

dans laquelle
-X- représente -C($R^4R^5$)- , -O- ou -S- ;
n vaut 2 ou 3 ;

$R^1$ représente H ou un groupe alkyle en $C_1$ à $C_4$;
$R^3$ représente H, un groupe halogéno, un groupe alkyle en $C_1$ à $C_4$, O(alkyle en $C_1$ à $C_4$), nitrile, phényle ou phényle substitué par un ou deux substituants choisis parmi un groupe alkyle en $C_1$ à $C_4$, O(alkyle en $C_1$ à $C_4$), S(alkyle en $C_1$ à $C_4$), halogéno, et phényle éventuellement substitué par un groupe alkyle en $C_1$ à $C_4$, O(alkyle en $C_1$ à $C_4$), S (alkyle en $C_1$ à $C_4$), ou halogéno ;
$R^4$ et $R^5$ sont choisis chacun indépendamment parmi H ou un groupe alkyle en $C_1$à $C_4$ ;
Ar- est choisi dans le groupe formé par

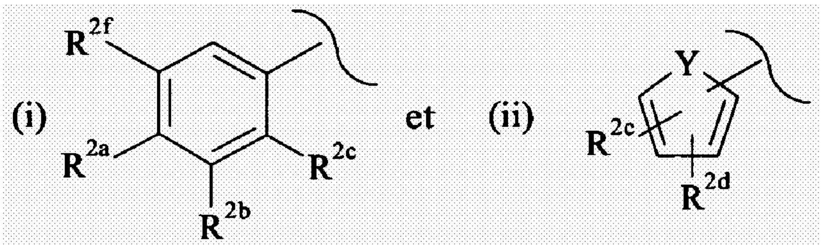

dans lesquelles
$R^{2a}$ représente H, un groupe halogéno, méthyle ou éthyle ;
$R^{2b}$ représente H, un groupe halogéno, ou méthyle ;
$R^{2c}$ représente H, un groupe halogéno, méthyle, trifluorométhyle, nitrile, ou méthoxy ;
$R^{2d}$ représente H, un groupe halogéno, méthyle ou éthyle ;
$R^{2c}$ représente H, un groupe halogéno, méthyle, trifluorométhyle, nitrile ou méthoxy ;
$R^{2f}$ représente H, ou un groupe fluoro ;
-Y- représente -O-, -S- ou -N($R^6$) ; et
$R^6$ représente H, ou un groupe méthyle
ou un sel de celui-ci, acceptable sur le plan pharmaceutique.

**2.** Composé selon la revendication 1, représenté par la formule (Ia)

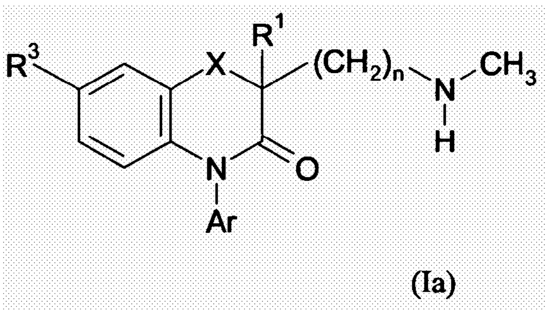

(Ia)

dans laquelle -X-, n, $R^1$, $R^3$ et Ar prennent les valeurs telles que définies pour la formule (I) dans la revendication 1.

**3.** Composé selon la revendication 1 ou 2, dans lequel -X-représente -C($R^4$ $R^5$)-.

**4.** Composé selon l'une quelconque des revendications 1 à 3, dans lequel Ar représente (i).

**5.** Composé selon l'une quelconque des revendications 1 à 3, dans lequel Ar représente (ii) et -Y- représente -S-.

**6.** Composé selon l'une quelconque des revendications 1 à 3, représenté par la formule (II).

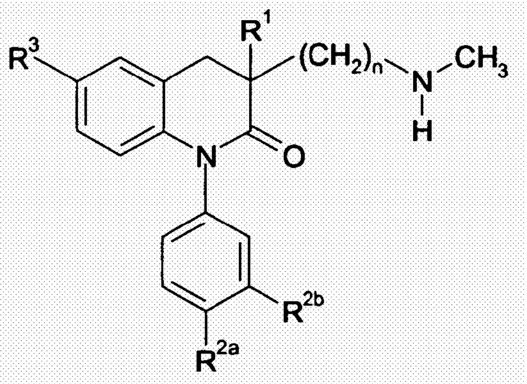

(II)

dans laquelle n, $R^1$, $R^{2a}$ et $R^{2b}$ prennent les valeurs telles que définies pour la formule (I) dans la revendication 1, et $R^3$ représente H, un groupe halogéno, phényle ou phényle substitué par un ou deux substituants choisis parmi un groupe alkyle en $C_1$ à $C_4$, O(alkyle en $C_1$ à $C_4$), S(alkyle en $C_1$ à $C_4$), halogéno, et phényle éventuellement substitué par un groupe alkyle en $C_1$ à $C_4$), O(alkyle en $C_1$ à $C_4$), S(alkyle en $C_1$ à $C_4$), ou halogéno.

**7.** Composé selon l'une quelconque des revendications 1 à 6, dans lequel $R^3$ représente H ou un groupe halogéno.

**8.** Composition pharmaceutique comprenant un composé selon l'une quelconque des revendications 1 à 7 ou un sel de celui-ci acceptable sur le plan pharmaceutique, conjointement avec un diluant ou véhicule acceptable sur le plan pharmaceutique.

**9.** Composé selon l'une quelconque des revendications 1 à 7, ou un sel de celui-ci acceptable sur le plan pharmaceutique, destiné à une utilisation en thérapie.

**10.** Utilisation d'un composé selon l'une quelconque des revendications 1 à 7 ou un sel de celui-ci acceptable sur le plan pharmaceutique, pour fabriquer un médicament destiné à traiter un trouble choisi dans le groupe formé par un trouble déficitaire de l'attention (ADD) dû à des états médicaux généraux, un trouble d'hyperactivité avec déficit de l'attention (ADHD), un trouble de la conduite, une dépression, un trouble oppositionnel avec défiance, et des troubles cognitifs dont le déficit cognitif léger (MCI), la démence du type Alzheimer (DAT), la démence vasculaire, et le déficit cognitif associé à la schizophrénie (CIAS).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5552429 A
- EP 0919236 A
- WO 0127068 A
- WO 02094262 A
- WO 0240006 A


### Non-patent literature cited in the description


- *J. of Medicinal Chemistry,* 1972, vol. 15 (7), 762-770
- **BUCHWALD.** *J. Am. Chem. Soc.,* 2001, vol. 123, 7727
- **T.W. GREENE.** Protective Groups in Organic Synthesis. John Wiley and Sons, 1991
- **BUCHWALD et al.** *Tetrahedron,* 1996, vol. 52, 7525
- **VANDENBERGH DJ ; PERSICO AM ; UHL GR.** A human dopamine transporter cDNA predicts reduced glycosylation, displays a novel repetitive element and provides racially-dimorphic TaqI RFLPs. *Molecular Brain Research,* 1992, vol. 15, 161-166
- **PACHOLCZYK T ; BLAKELY, RD ; AMARA SG.** Expression cloning of a cocaine- and antidepressant-sensitive human noradrenaline transporter. *Nature,* 1991, vol. 350, 350-354
- **RAMAMOORTHY S ; BAUMAN AL ; MOORE KR ; HAN H ; YANG-FENG T ; CHANG AS ; GANAPATHY V ; BLAKELY RD.** Antidepressant- and cocaine-sensitive human serotonin transporter: Molecular cloning, expression, and chromosomal localization. *Proceedings of the National Academy of Sciences of the USA,* 1993, vol. 90, 2542-2546
- **MOODY GC ; GRIFFIN SJ ; MATHER AN ; MCGINNITY DF ; RILEY RJ.** Fully automated analysis of activities catalyzed by the major human liver cytochrome P450 (CYP) enzymes: assessment of human CYP inhibition potential. *Xenobiotica,* 1999, vol. 29 (1), 53-75